# EUROPEAN PATENT APPLICATION

(11) **EP 4 116 710 A1**
(43) Date of publication of application: **11.01.2023**
(21) Application number: 21765474.8
(22) Date of filing: 08.03.2021
(51) Int. Cl.: G01N 33/50, A61K 31/192, A61P 1/16

(54) **USE OF A GROUP OF MARKERS FOR DIAGNOSING AND ADJUSTING TREATMENT OF PRIMARY BILIARY CHOLANGITIS, PHARMACEUTICAL COMPOSITION AND SOLID DOSAGE FORM FOR TREATING PRIMARY BILIARY CHOLANGITIS**

(30) Priority: 06.03.2020 UA 202001611 U; 06.03.2020 UA 202001613 U; 05.03.2021 UA 202101117
(71) Applicant: Mili Healthcare Trade DMCC, Limited Liability Company, Dubai (AE)
(72) Inventor: JAIN, Nitin, Delhi 110092 (IN)
(74) Representative: Lichtnecker, Markus Christoph
(86) International application number: PCT/IB2021/051918
(87) International publication number: WO 2021/176430

(57) **Abstract**

The present disclosure relates to the field of medicine, in particular, to the methods and means for the treatment of primary biliary cholangitis.

A solid dosage form for the treatment of primary biliary cholangitis, containing ursodeoxycholic and obeticholic acids, are provided.

The technical contribution resides in obtaining a new all-purpose solid dosage form for the treatment of primary biliary cholangitis, which comprises both ursodeoxycholic and obeticholic acids, which is effective in use at all stages of primary biliary cholangitis and is **characterized by** a complex mechanism of action. In particular, simultaneous blockage of the transport and synthesis of bile acids is achieved.

## Description

The present disclosure relates to the field of medicine, in particular, to the methods and means for the treatment of primary biliary cholangitis.

Primary biliary cholangitis (PBC), formerly known as primary biliary cirrhosis, is the most common autoimmune liver disease. This disease results in a gradual destruction of the small bile ducts of the liver, which leads to the accumulation of the bile in the liver tissues (a condition called cholestasis). Over time, the accumulated bile starts to destroy the liver, leading to fibrosis and cirrhosis. The risk group is middle-aged women, wherein the ratio of suffering women to men ranges from 1.6 to 10.11-14, although higher mortality rates have been recorded for men.

At first, PBC was considered a very rare disease, due to the small sample size and lack of the large population-based studies. Moreover, PBC is unevenly distributed throughout different regions of the world. The highest prevalence rates were recorded in China, the USA, Greece, and England (49.2; 40.2; 36.5 and 24.0 cases per 100,000 people, respectively), while Canada and Australia had the lowest number of the cases (2.2 and 1.9 cases, respectively). However, the overall incidence of PBC in the world has continued to rise since the 1980s.

In Ukraine, there are currently no longitudinal studies of the epidemiology of PBC. Studies conducted in Europe and Asia generally report a steady increase in the prevalence of the disease over the past decade. In the USA, over a 12-year period, the prevalence of PBC increased by more than 72% in women (from 33.5 to 57.8 per 100,000 people), and by more than 114% in men (from 7.7 to 15.4 per 100,000 people). The Hong Kong Epidemiology Study found that the age/sex-adjusted medium annual incidence rate increased from 6.1 to 8.1 per million person-years, and the age/sexadjusted prevalence rate increased from 31.1 to 82.3 per million person-years between 2000 and 2015. In Sweden, a large-scale population-based analysis of inpatient and outpatient registries showed that the prevalence of PBC steadily increased from 5.0 to 34.6 per 100,000 people from 1987 to 2014, respectively.

The causes and factors leading to the development of PBC are unknown. One of the determinant factors for the development of PBC is the loss of immune tolerance to the autoantigen of the PDC-E2 pyruvate dehydrogenase complex. The PDC-E2 complex plays a fundamental role in activating the cellular response of Th1 T-helpers. Th1-cells start to produce INF-y interferon gamma and TNF-α tumor necrosis factor, that have a cytotoxic effect.

The abnormal destruction of the liver cells (hepatocytes) and bile duct epithelial cells (cholangiocytes) in PBC may be explained by the hypothesis of bicarbonate (HCO₃⁻) umbrella loss, which is supported by experimental, clinical, and genetic studies. The hypothesis is based on the statement that, by secreting a bicarbonate anion (HCO₃⁻) into the lumen of the bile duct, cholangiocytes and hepatocytes create a protective apical alkaline barrier that stabilizes glycocalyx. This alkaline barrier also preserves bile acids in the form of polar, water-soluble conjugates (often also referred to as bile salts) that are unable to cross cell membranes. In PBC, the functioning of the transporters and channels located on the apical and basolateral membranes of cholangiocytes involved in the formation and export of HCO₃⁻ ions is disrupted. This results in weakening of the alkaline barrier, which leads to partial protonation of the bile acid conjugates, as a result of which they become apolar and acquire the ability to cross the cholangiocyte membrane, regardless of the activity of the transporters, and induce apoptosis in cholangiocytes and hepatocytes.

Recent studies have shown that the POGLUT1 gene plays an important role in pathogenesis of this disease. Moreover, the tendency to develop PBC is associated with polymorphism in the human leukocyte antigen (HLA) region, especially in the DRB1*08 DRB1*11, DRB1*14 DPB1*03: 01 and DQB1 alleles.

Additionally, over the past decades, a correlation between the etiopathogenesis of the disease, environmental factors, and impaired immune tolerance in PBC has been observed. In this regard, the role of pathogens of various infections in the etiology of PBC has been hypothesized. For example, patients with PBC have a higher prevalence of urinary tract infections, mainly associated with E. coli. It has also been shown that the gram-negative microorganism Novosphingobium aromaticivorans can be an etiological factor in the development of PBC. The theoretical mechanism is the cross-reactivity of antibodies against the surface proteins of the microorganism with mitochondria of hepatocytes.

In addition, the gut microflora can also influence PBC, since the presence of metabolic byproducts of microorganisms, such as Toll-like receptor (TLR) ligands and CpG motifs can contribute to the development of an autoimmune response of the human body.

The action of xenobiotics can also influence pathobiology of the liver and stimulate local immune responses, since the liver is the main organ responsible for the chemical detoxification of xenobiotics. For example, it was reported that several groups of the patients diagnosed with PBC lived in regions that were located near supertoxic waste storage sites. Although controversial, some epidemiological studies have correlate cosmetics use and smoking to the development of PBC. Specific environmental factors that may lead to loss of the tolerance to PDC-E2 have been found. They are xenobiotics that can either mimic or modify lipoic acid. These include 2-octynic acid and 6,8-bis-acetylthiooctanoic acid (a metabolite of acetaminophen), which are often used in cosmetics.

Moreover, gamma radiation can also cause autoimmune reactions, since a high number of PBC cases have been found among survivors of the Nagasaki nuclear disaster.

PCB often co-occurs with other autoimmune diseases, including Sjögren's syndrome and chronic thyroiditis.

However, PBC is now regarded as a multifactorial polygenic disease, in which impaired immune tolerance is explained not only by environmental factors, but also by genetic susceptibility and epigenetic phenomena that strongly contribute to the pathogenesis of the disease.

The importance of genetic factors in the development of PBC has been shown in multiple studies. A study on the pathogenesis of PBC in twins that had been conducted over the past decade has shown that the incidence rate is higher in monozygotic twins than in dizygotic twins (with a pairwise concordant ratio of 0.63, which is one of the highest ratios for autoimmune diseases), indicating high genetic susceptibility for the development of the disease.

The process of the PBC development can be divided into the following stages:
Stage 1 - a trigger that initiates liver cells damage.
Stage 2 - the development of cholestasis and induction of inflammation.
Stage 3 - the development of high intensity inflammation.
Stage 4 - fibrosis.
Stage 5 - cirrhosis.

Explanations in more details are given below.

### 1) A trigger that initiates liver cells damage. For PBC, this is an autoimmune process.

Damage to the liver cells onsets with an autoimmune response of the body to mitochondrial and nuclear antigens. The most common autoimmune reaction is the synthesis of antimitochondrial antibodies (AMA). AMAs have specificity against members of the family of multienzymatic 2-oxo-acid dehydrogenase (2-OADC) complexes. These complexes, which play an important role in the energetics of mitochondria, have a common multicomponent multidomain structure.

### 2) Development of cholestasis and induction of inflammation.

Cholestasis (cholestatic syndrome) is an impairment of the processes of the bile synthesis and/or bile excretion, resulting in a decrease in the amount of the bile that enters the duodenum. Cholestasis can be asymptomatic or manifest as fatigue, itching in the right upper abdomen, often as so-called cholestatic / obstructive jaundice.

### 3) The development of high intensity inflammation.

Inflammation is an important mechanism for the PBC progression. A high concentration of toxic lipids, mainly free fatty acids (FA), causes cellular stress and induces specific signals that trigger hepatocyte apoptosis, which is the main mechanism of the cell death in PBC, and correlates with the degree of liver inflammation and fibrosis. Various types of the human immune cells, such as monocytes, macrophages, and others, also migrate to the site of inflammation contributing to the development and progression of PBC.

### 4) Fibrosis.

Chronic inflammation and damage to the liver cells leads to the development of the fibrotic processes, as a result of which the connective tissue replaces the normal liver parenchymal tissue and forms scars. Growth factors, cytokines and chemokines secreted by activated macrophages play a central role in the activation of fibrosis. Connective tissue is formed by the production of a huge amount of extracellular matrix proteins (mainly collagen I and III) caused by monocyte-derived macrophages, resident macrophages, as well as damaged hepatocytes, hepatic stellate cells (HSC) and some other cell types. This results in the replacement of the normal liver tissues with the scar tissue and development of circulatory disorders inside the organ. The development of liver fibrosis leads to significant changes in the quantity and quality of the hepatic extracellular matrix, impaired liver detoxification function, liver failure and, at the last stage, liver cirrhosis.

### 5) Cirrhosis.

Cirrhosis occurs when the liver undergoes significant damage as a result of the fibrosis progression. The scar tissue gradually replaces the normal liver tissue, blocks portal blood flow (resulting in increased pressure in the portal vein and portal hypertension development) and leads to the inability of the liver to perform its functions. Liver cirrhosis progresses over time with manifestation of the decompensation symptoms. The mean survival rate of the patients with liver cirrhosis after the first 5 years from the onset of the first symptoms of decompensation is 45%, after 10 years - only 10-20%. The main treatment for decompensated cirrhosis is liver transplantation.

In rare cases, PBC is complicated by hepatocellular carcinoma - HCC (approximately 2-2.5% of all cases of PBC incidence). The results of epidemiological studies of the correlation between PBC and HCC are generally consistent with the idea that liver cirrhosis is a risk factor for the development of HCC. It is not currently possible to determine whether there are any other PBC-specific risk factors for the development of HCC other than cirrhosis.

Primary biliary cholangitis has no specific symptoms, so it is very difficult to be detected in its early stages. Usually the patients experience tiredness (up to 80% of cases), depression. In some cases, are developed hypothyroidism, anemia, obesity, dry skin and eyes. Itching, usually mild to moderate in intensity, occurs in 20-70 % of the patients. Itching correlates with general fatigue and negatively affects night sleep, which can significantly reduce the patient's quality of life.

In later stages of the disease, the following symptoms may develop:
- nausea;
- abdominal pain;
- loss of appetite;
- weight loss;
- joint pain;
- jaundice;
- xanthoma and/or xanthelasma;
- accumulation of ascites in the abdominal cavity;
- systemic edema.

Without appropriate treatment, PBC leads to chronic inflammation, fibrosis, and as a result, liver cirrhosis. There is no specific treatment for PBC. Treatment is aimed at stopping the progression of the disease and eliminating symptoms.

Currently, due to non-specific symptoms, or asymptomatic course of the disease, in the early stages PBC is most often diagnosed incidentally during a general physical examination. In people with suspected PBC, the diagnosis is based on biochemical methods, such as determination of the levels of ALT (alanine aminotransferase) and AST (aspartate aminotransferase) liver enzymes, as well as total bilirubin, antimitochondrial (AMA) and antinuclear (ANA) antibodies in the blood serum. Sometimes additional non-invasive research methods (ultrasound, MRI, etc.) are carried out. In rare cases, a biopsy may be taken. A high titer of AMA is observed in more than 95% of the patients with PBC.

However, determining concentration level values of these markers is not enough to establish an accurate clinical picture for the patient with PBC, because they are superficial and do not reflect the processes occurring in the liver at the cellular and molecular level. Levels of AMA/ANA markers indicate the presence or absence of the autoimmune processes. The level of liver enzymes enables establishing only the fact of the liver cell destruction. Generally, said markers enable determining only the stage of cirrhosis and the general condition of the liver. Thus, based on the imperfect results of the study, it is impossible for the physician to determine the stage of PBC and prescribe an effective treatment to cease its development, especially in the early stages of PBC (stages 1-2).

To date, PBC is an incurable disease with unknown mechanisms of onset. Since PBC is a chronic, progressive disease, current treatments focus only on arresting the progression of the disease and relieving symptoms. Essentially, an effective treatment or an adequate patient response to the treatment means that a suppression in the PBC progression has been achieved. Without treatment, PBC leads to chronic inflammation, fibrosis, and, eventually, liver cirrhosis. Current protocols for the PBC treatment include ursodeoxycholic acid (UDCA) pharmaceutical compositions and obeticholic acid (OCA) pharmaceutical compositions.

For example, there is a known pharmaceutical composition of the medicinal product URSO 250^{®} for the treatment of primary biliary cholangitis in the solid dosage form of a coated oral tablet comprising 250 mg of ursodeoxycholic acid per tablet, microcrystalline cellulose, povidone, sodium starch glycolate, magnesium stearate, ethylcellulose, dibutyl sebacate, carnauba wax, hydroxypropyl methylcellulose, PEG 3350, PEG 8000, cetyl alcohol, sodium lauryl sulfate and hydrogen peroxide. (https://www.drugs.com/cdi/urso-250.html) .

UDCA, and thus URSO 250^{®}, is the first-line drug prescribed for all patients with diagnosed PBC, regardless of the disease stage, with exception of decompensated cirrhosis (one of the few contraindications for UDCA use). The recommended daily dose of the known pharmaceutical composition is 13-15 mg of ursodeoxycholic acid per 1 kg of the patient body weight, divided into 2 or 4 consumptions with food (4-5 tablets per day based on 80 kg of the patient body weight). The daily dose may be adjusted by the physician according to the patient condition.

The first stage of PBC treatment continues for 1 year, after which the patient biochemical response to UDCA is determined and the effectiveness of the treatment is evaluated.

Effectiveness evaluation is carried out using various evaluation systems, such as Paris, Paris2, Rotterdam, etc. For example, according to the Paris2 system, the criteria for the satisfactory treatment of PBC are:
- ALT level ≤ 3-fold upper limit of normal (ULN);
- AST level ≤ 2-fold ULN;
- normal bilirubin level.

The effectiveness of the PBC treatment depends on many factors, the main being the stage of the disease development. Patient age and gender have also been shown to influence response and outcomes of the PBC treatment. Younger patients (under 45 years) tend to have symptomatic PBC and are less likely to respond to standard UDCA therapy. As a result, this population has a higher standardized mortality rate, in particular, there are more deaths due to liver disorders, while older people are more likely to die from non-liver causes. Male sex is characterised with a delayed time of diagnosis, a poorer biochemical response to UDCA therapy, and a greater risk of developing hepatocellular carcinoma.

According to statistics, only for 60% of the patients the treatment of PBC with pharmaceutical compositions containing UDCA is successful (i.e., there is a suspension of the PBC development). The other 40% of the patients with an inadequate response to UDCA treatment (when PBC continues to progress, and the patient condition worsens) are treated with OCA pharmaceutical compositions.

For example, there is a known pharmaceutical composition for the treatment of primary biliary cholangitis called OCALIVA^{®} in the solid dosage form of a coated oral tablet containing 5 or 10 mg of obeticholic acid per tablet, microcrystalline cellulose, sodium starch glycolate, magnesium stearate, partially hydrolyzed polyvinyl alcohol, titanium dioxide, macrogol, talc, and iron oxide yellow (https://www.accessdata.fda.gov/drugsatfda_docs/label/2018/207999s0031bl.pdt).

Before starting OCALIVA^{®} in the patients with suspected liver cirrhosis, a nomogram should be used to determine the cirrhosis stage according to the Child-Pugh system (A, B or C) and establish the appropriate initial dose of the pharmaceutical composition. The recommended starting dose and titration regimen for OCALIVA^{®} in the patients who have not achieved an adequate biochemical response to an appropriate dose of UDCA (high-risk group) for at least 1 year, or who are intolerant to UDCA, depends on the disease stage.

For the patients, who have not yet developed cirrhosis or with type A compensated liver cirrhosis, OCALIVA^{®} is prescribed for the first 3 months in an amount corresponding to 5 mg of obeticholic acid per day. If after 3 months the patient has not achieved an adequate decrease in alkaline phosphatase (AP) and/or total bilirubin, the daily dose is increased to 10 mg of obeticholic acid (providing that the patient tolerates it well). The maximum daily dose of OCALIVA^{®} for this category of the patients is 10 mg of obeticholic acid. Before adjusting the dose, it is recommended to recalculate the dose according to the Child-Pugh system.

For the patients with type B or C compensated liver cirrhosis or with a previous case of decompensation (gastroesophageal variceal bleeding, new case or worsening of jaundice, spontaneous bacterial peritonitis, etc.), during the first 3 months, OCALIVA^{®} is prescribed in an amount corresponding to 5 mg of obeticholic acid per week. If after 3 months the patient has not achieved an adequate reduction in alkaline phosphatase and/or total bilirubin, the dose is increased to 5 mg of obeticholic acid twice a week, with an interval between doses of at least 3 days, with a possible titration to 10 mg of obeticholic acid twice a week, with an interval between doses of at least 3 days. Dosage depends on the response and condition of the patient. The maximum dose of OCALIVA^{®} for this category of the patients is 10 mg of obeticholic acid per week, with an interval between doses of at least 3 days. Before adjusting the dose, it is recommended to recalculate the dose according to the Child-Pugh system.

The use of current treatment protocols and known URSO 250^{®} and OCALIVA^{®} for the PBC treatment leads to a large number of negative consequences.

The main disadvantage is that, due to inability to establish the disease stage and utilization of ineffective outdated markers, physicians cannot prescribe an effective treatment.

Therefore, the available protocols for the PBC treatment are based on the outdated markers (ALT, AST, bilirubin), that do not identify the disease by stages, but identify only the presence or absence of the disease. The available protocols require the use of the pharmaceutical compositions based on ursodeoxycholic or obeticholic acid. However, a year is wasted trying to apply UDCA and staging PBC.

Moreover, the initial stages of PBC are almost never identified. The use of the current research protocols and markers enables diagnosing PBC only in the later stage when the disease progresses over time and begins to manifest itself.

Starting from the second stage of PBC, it is no longer advisable to waste time treating the patient with the UDCA pharmaceutical compositions, since the percentage of the development and transition to irreversible stages of the disease is high. Therefore, an accurate and timely diagnosis of the PBC stage will result in the physician being able to prescribe safer and more effective treatment.

The therapeutic factor is the main disadvantage. OCALIVA^{®} has received fast-track USFDA approval for the treatment of PBC, confirming the great need for new pharmaceutical compositions and methods of PBC treatment. In particular, such a need remains very urgent given the large percentage (40%) of the patients with an inadequate response to the treatment with ursodeoxycholic acid pharmaceutical compositions, such as URSO 250^{®}. However, the current protocols for the PBC treatment involve using the obeticholic acid pharmaceutical compositions only after the end of the first stage of the treatment, which continues for 12 months and includes the use of UDCA pharmaceutical compositions. Over such a long period of time, the patients with an inadequate response to UDCA treatment achieve no therapeutic effect. PBC not only does not stop its development, but also continues to progress, transit to more serious stages, that are much more dangerous for patients and significantly worsen their quality of life. At the last stages of the PBC development, cirrhosis manifests itself, which significantly reduces the survival rate of the patients, even after liver transplantation.

The second disadvantage is compliance. The patient compliance with all physician recommendations on the treatment regimen is a very important factor for the treatment of chronic diseases such as PBC. To achieve positive therapeutic results, the patient must follow many rules, such as taking medicines on time and in the required dose, following the physician recommendations regarding lifestyle and nutrition, and maintaining a positive psychological (emotional) condition. The patient propensity for the treatment, or compliance, depends on many factors:
- age, emotional condition, general level of education;
- the number of the pharmaceutical compositions to be taken and the frequency of their intake per day;
- the number of the tablets to be taken per day;
- a form of the pharmaceutical composition that is inconvenient to use or causes discomfort to the patient;
- the rate of achieving a therapeutic effect;
- insufficient level of information about the disease and/or pharmaceutical composition;
- restrictions in the usual lifestyle;
- side effects from the use of the pharmaceutical compositions;
- costs of the purchase of the pharmaceutical compositions.

Considering the current situation regarding the PBC treatment protocols, it can be concluded that patients suffering from PBC are characterized by low compliance, thus, the known pharmaceutical compositions for the treatment of PBC, such as URSO 250^{®} and OCALIVA^{®}, also have poor compliance. This is due to several reasons.

First, adherence to the treatment regimen may be difficult for the elderly, who find it troublesome to keep track of the number of the doses and dosing of the pharmaceutical composition.

Secondly, the first stage of the PBC therapy involves long-term daily use of the pharmaceutical compositions with high doses of UDCA (up to 4 doses per day). At the same time, the therapeutic effect of such pharmaceutical compositions, for example, URSO 250^{®}, does not appear quickly, but along with the accumulation of the active substance in the patient body. Given the nature of the disease and current methods of its treatment, the positive results of the PBC treatment can often be determined only by a medical expert. Moreover, in 40% of cases of using the pharmaceutical compositions containing UDCA, an adequate response to the treatment is not observed at all, and the patient condition worsens even more. This significantly undermines the patient confidence in the physician, the therapy regimen and pharmaceutical compositions prescribed, and reduces the incentive for further treatment.

Another disadvantage of such long-term use of the pharmaceutical compositions is the change in the regimen in accordance with the disease stages. The PBC treatment is a long process, extended over years. At the same time, the emotional condition of the patients is rather unstable, they experience constant fatigue and irregularity of behavior, therefore, when the patient gets used to particular algorithm for taking medications, it is desirable for them to keep this algorithm during further treatment. Today, there is the treatment with several pharmaceutical compositions that alternate in time, while the pharmaceutical composition which regimen does not need to be changed (only the dose changes) will have a much greater compliance.

Another disadvantage is the economic factor. During the first stage of the PBC treatment, which continues for 12 months, the patient spends money on ursodeoxycholic acid pharmaceutical compositions, although, after completion of the first stage of the PBC treatment, almost in half of the patients UDCA pharmaceutical compositions are recognized to be ineffective.

Considering the above reasons, there is a high probability that the patient suffering from PBC will not appropriately follow the physician recommendations for the treatment of PBC, as a result of which the effectiveness of the disease treatment will be significantly less.

Thus, the current situation is as follows: the molecular mechanisms of the PBC development are not studied, the disease stages are not diagnosable, PBC is almost never diagnosed in the early stages, therefore, the PBC therapy is now carried out simply upon identification of the disease or detection of liver cirrhosis. Whatever stage of PBC the patient has at the start of the treatment, they are treated first with UDCA pharmaceutical compositions for a year, only then consideration is given to OCA pharmaceutical compositions at a dose of either 5 mg/day, with a possible dosage increase to 10 mg/day, or, in the presence of decompensated cirrhosis of B and C stages, 5 mg or 10 mg, but in a week. During the first year of the treatment with UDCA pharmaceutical compositions, which is spent on diagnosing and dividing patients into low-risk and high-risk groups, in 40% of the patients, the PBC progression not only does not stop, but continues, and transits to more severe and irreversible stages of the development. Thus, the first year is no less significant than the process of diagnosing the disease as a whole and should be spent not on clarifying the diagnosis and selecting treatment, but directly on the adequate treatment. Other disadvantages are as follows:
- The onset of PBC - the first and second stages are not diagnosable today.
- Postponed diagnosis and, accordingly, treatment of PBC for a year.
- The effectiveness of UDCA treatment is only 60%.
- The molecular mechanism of the disease is unknown.
- The stages of the disease are not diagnosable (except for cirrhosis).
- Impossibility of prescription of the effective treatment from the first visit to the physician.
- Lack of the pharmaceutical composition for the treatment of all stages of PBC.

Thus, now there is a need for a global change in the principles and approaches to the diagnosis and treatment of PBC.

### SUMMARY OF THE INVENTION

The objective technical problems to be solved are:
- increase of the percentage of patients for whom PBC treatment will be effective;
- development of a more efficient way for the differentiation of PBC with division into stages of the disease;
- development of a more efficient way for the diagnosis and adjustment of the PBC treatment including early stages of PBC (stages 1 and 2);
- determination of the precise and effective treatment regimen for the patients with PBC according to the stage of the disease;
- reduction of the first stage of the treatment, in which the patients are divided into groups, from one year to 1-2 weeks;
- development of a new pharmaceutical composition for the PBC treatment, which is more effective for the PBC treatment and can be used in all stages of the disease, has better compliance and improves the patient's quality of life;
- development of a new pharmaceutical composition that would increase the effectiveness of the PBC treatment from 60% to more than 80% with the possibility of use in all stages of the disease;
- development of a new pharmaceutical composition with a structural solution that prevents the competition of two active pharmaceutical ingredients (APIs) for the transporters.

The objective technical problems are solved by developing a new group of the markers for the PBC diagnosing and differentiating the disease on stages, followed by the dividing the patients for the targeted treatment.

In the present disclosure, the authors selected and studied the new group of the additional markers and the use of the new group of the additional markers, that was selected and developed for the diagnosis of the PBC stages and can diagnose the disease in the early stages. In addition, the new group of the markers enables studying the molecular processes of the disease course and demonstrates the need to simultaneously stop the synthesis of the bile acids (BA) and stimulate the BA transport from the very first stage of PBC. Thus, there is a need to take a pharmaceutical composition that comprises both OCA and UDCA. In the first stage of the disease, the use of UDCA may still be appropriate, but, starting from the second stage, the simultaneous use of OCA and UDCA becomes effective. Moreover, OCA inhibits the synthesis of fatty acids, and UDCA removes them from the body. But, to date, this was not possible, since there are no studies on how to identify the stages of PBC, there are no treatment protocols and, accordingly, there are no pharmaceutical compositions comprising a mixture of both acids - OCA and UDCA. That is, when using the new group of the markers and dividing the patients into groups in accordance with the treatment regimens, it becomes possible for them to switch to the treatment with the mixture of acids, which leads to an increase in the effectiveness of the PBC treatment, namely, to more than 80% of patients with adequate response to the treatment. This result is achieved precisely through the mechanism of action of two acids known for the treatment of this disease, however, in the specific stage of PBC, and without at least one-year-delay from the PBC diagnosing, and their use in one solid dosage form. This becomes possible exactly because of the identification of molecular processes in the liver in PBC and the determination of the disease stage by using the new group of the additional markers, such as IL-6, Nf-kB, MCP-1/CCL2, and Bcl-2. Due to this new disclosure, it becomes possible to adjust the existing treatment protocols and make the PBC treatment more effective by determining the treatment regimen and dividing the patients according to the marker concentration values and treatment regimens, and to increase the number of the patients with a positive response to the treatment with a pharmaceutical composition comprising two APIs - obeticholic acid and ursodeoxycholic acid, to more than 80%.

The objective technical problem is solved by the fact that the additional markers, chosen by the authors, enable diagnosing PBC in the early stage. In the case where AMA/ANA antibodies have already been diagnosed, but liver tests are normal (stages 1 and 2 of PBC), PBC is not currently diagnosable, and no treatment is provided. And when the additional markers according to the invention are prescribed, the presence or absence of the disease will be identified and, accordingly, the treatment regimen will be prescribed. The authors investigated and divided the PBC stages according to the marker concentration values.

The present invention relates to a solid dosage form comprising ursodeoxycholic acid and at least one excipient for use in treating primary biliary cholangitis, wherein it additionally comprises obeticholic acid, wherein solid dosage form comprises the core comprising ursodeoxycholic acid, and at least one layer comprising obeticholic acid, and is a modified release dosage form..

In one aspect, the mass ratio of ursodeoxycholic acid to obeticholic acid ranges from 15:1 to 750:1.

In one aspect, the solid dosage form comprises three layers and the core.

In one aspect, the solid dosage form comprises an outer layer, a second layer comprising obeticholic acid, a third layer, and a core comprising ursodeoxycholic acid.

In one aspect, the outer layer is an enteric coating or a gastric acid soluble coating.

In one aspect, the second layer comprises obeticholic acid in an amount of 1-50 mg.

In one aspect, the second layer comprises obeticholic acid in an amount of 1-15 mg.

In one aspect, the third layer is a STOP layer with a delayed dissolution or an enteric coating.

In one aspect, the dissolution time of the STOP layer is 1-2 hours.

In one aspect, the core comprises ursodeoxycholic acid in an amount of 100-1000 mg.

In one aspect, the core has a modified release of ursodeoxycholic acid within 1-6 hours.

In one aspect, the solid dosage form comprises the outer layer being the enteric coating, the second layer comprising obeticholic acid, the third layer being STOP layer, and the core comprising ursodeoxycholic acid.

In one aspect, primary biliary cholangitis is characterised by the following concentration values of the IL-6, Nf-kB, MCP-1/CCL2, Bcl-2, TNF-α and MDA markers in the patient sample:

| | |
|---|---|
| IL-6 | - at least 3.0 pg/ml; |
| Nf-kB | - at least 14.0 pg/ml; |
| MCP-1/CCL2 | - at least 215 pg/ml; |
| Bcl-2 | - at least 0.24 U/ml; |
| TNF-α | - at least 0.14 hg/ml. |
| MDA | - at least 4.3 nmol/ml |

In one aspect, the solid dosage form comprises the outer layer being the gastric acid soluble coating, the second layer comprising obeticholic acid, the third layer being enteric coating, and the core comprising ursodeoxycholic acid.

In one aspect, primary biliary cholangitis is characterised by the following concentration level values of the markers IL-6, Nf-kB, MCP-1/CCL2, Bcl-2, TNF-α and MDA in the patient sample:

| | |
|---|---|
| IL-6 | - at least 3.0 pg/ml; |
| Nf-kB | - at least 14.0 pg/ml; |
| MCP-1/CCL2 | - at least 215 pg/ml; |
| Bcl-2 | - at least 0.24 U/ml; |
| TNF-α | - at least 0.14 hg/ml. |
| MDA | - at least 4.3 nmol/ml |

### BRIEF DESCRIPTION OF DRAWINGS

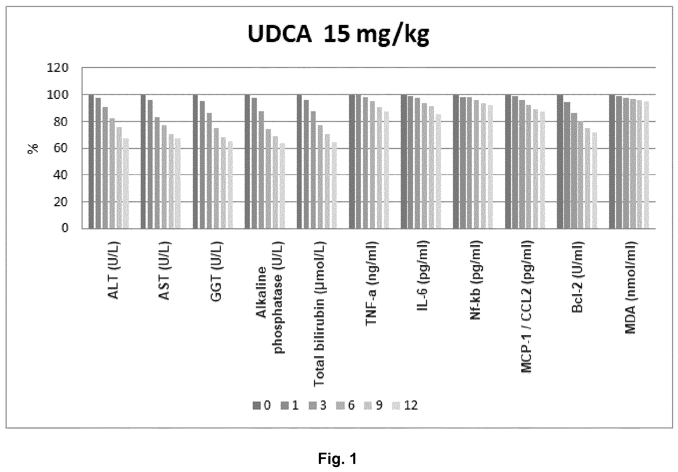

Fig. 1 shows a graphical representation of the changes in the marker concentration values in % of the initial value when using the standard treatment regimen No. 1 (UDCA 15 mg/kg).

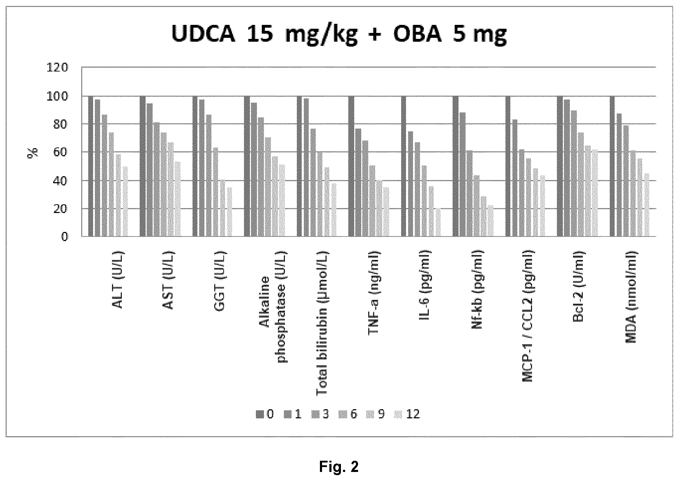

Fig. 2 shows a graphical representation of the changes in the marker concentration values in % of the initial value when using the provided treatment regimen No. 2 (UDCA 15 mg/kg + OCA 5 mg).
Fig. 3 shows a graphical representation of the changes in the marker concentration values in % of the initial value when using the provided treatment regimen No. 3 (UDCA 15 mg/kg + OCA 10 mg).

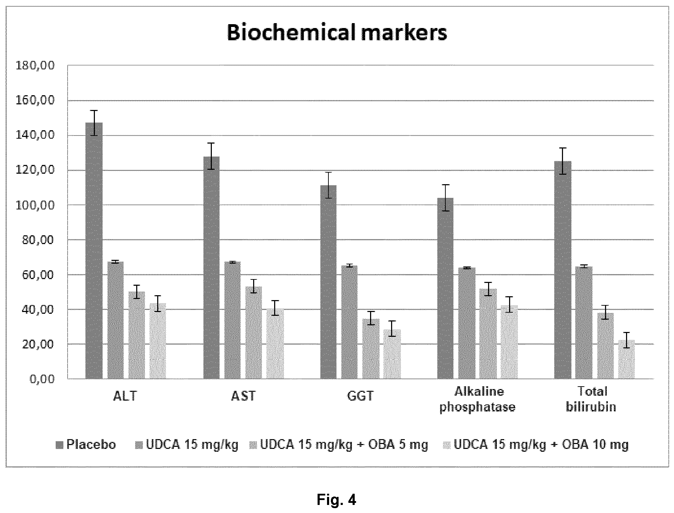

Fig. 4 shows the biochemical markers concentration values.

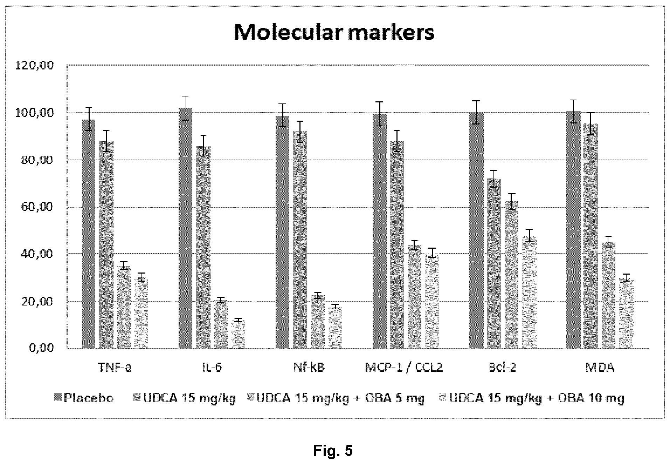

Fig. 5 shows the molecular markers concentration values.

Since the mechanism of the PBC onset is unknown and it is impossible to clearly diagnose PBC with the definition of the disease stage, the current treatment protocols involve the use of UDCA pharmaceutical compositions, that promote the elimination of FA. Ursodeoxycholic acid is indeed responsible for the transport of FA but does not stop their synthesis. In addition, studies conducted by the authors using the additional group of the markers to determine the processes of inflammation and identify apoptosis and fibrosis in PBC, show that starting from the second stage of PBC, UDCA is no longer effective, since it is already necessary to stop the FA synthesis. Thus, in the absence of the studies on the molecular mechanism and diagnosis of PBC, it was not possible and appropriate to use the pharmaceutical composition that comprises both acids - UDCA and OCA.

Because compliance is especially important for the PBC patients (as described above), this objective is achieved by developing a solid dosage form for the PBC treatment that comprises the pharmaceutical composition with UDCA and OCA.

Having conducted their own research, both theoretical and experimental, the authors of the present disclosure concluded that the system for diagnosing and treating PBC, which is used currently, has many disadvantages, and needs to be modified.

Specifically, the use of the new group of the markers for the diagnosis and adjustment of the PBC treatment, that includes the markers, such as IL-6, Nf-kB, MCP-1/CCL2, and Bcl-2, was provided.

IL-6 (Interleukin 6) is a pro-inflammatory interleukin synthesized by immune cells. It can stimulate the inflammatory process at the site of synthesis. An increase in the amount of IL-6 is observed in the early stages of the disease, when the autoimmune process is just beginning to develop. Its detection in liver biopsy specimens indicates an acute inflammatory process.

Nf-kB (nuclear factor kappa-light-chain-enhancer of activated B cells) is a protein complex that controls DNA transcription, cytokine synthesis and cell survival. NF-kB is found in almost all types of the animal cells and is involved in cellular responses to stimuli, such as stress, cytokines, free radicals, heavy metals, ultraviolet radiation, lipid peroxidation, and bacterial or viral antigens. NF-kB plays a key role in regulating the immune response. Misregulation of NF-kB is associated with the development of oncology, inflammatory and autoimmune diseases.

MCP-1/CCL2 (MCP-1, Monocyte chemoattractant protein-1) is one of the key chemokines that regulates the migration and infiltration of monocytes from peripheral blood into tissues. In PBC, it is released by Kupffer cells, and it stimulates the migration of monocytes from the peripheral blood to the liver tissue, which leads to further development of inflammation.

Bcl-2 is a mitochondrial apoptosis factor. Its elevated value indicates pathological cell death. This pathological phenomenon can be eliminated by neutralizing the primary cause of the cell death (in this case, this is the toxic effect of the bile acids and the autoimmune process).

In addition to the above markers, TNF-α and MDA markers can also be studied.

TNF-α (tumor necrosis factor alpha) is a cytokine, i.e., a small protein used by the immune system for signaling. The primary role of this molecule is the regulation of the activity and metabolism of the immune cells. It can induce inflammation and apoptosis, which may be the cause of many autoimmune diseases. In PBC, an increase in the value of this cytokine is one of the early events; when immune cells are just starting to attack their own cells, its value rises. This happens much earlier than the rise in ALT/AST and bilirubin levels. That is, the detection of this marker in liver biopsy specimens indicates the beginning of the development of the inflammatory process.

MDA is a marker of inflammation, but from the lipid peroxidation point of view. Its increase indicates a high level of highly reactive oxygen in the tissues, which can be caused by both the inflammation and immune response.

The new group of the markers is provided to be used in the diagnosis of PBC in the following way. The patient with suspected PBC is tested for AMA and ANA levels to confirm the presence of the autoimmune processes and establish the PBC. Based on the concentration values of the claimed group of the IL-6, Nf-kB, MCP-1/CCL2, and Bcl-2 markers (potentially additionally TNF-α and MDA), the stage of PBC is determined. Based on the established stage of PBC, the patient is prescribed specialized treatment. Next, the patient condition is monitored with a possible adjustment of the therapy regimen by the physician.

As a result of the studies, a scheme for determining the PBC stage was developed based on the concentration values of the claimed group of the markers. Concentrations of the TNF-α , IL-6, Nf-kB, MCP-1/CCL2, Bcl-2 and MDA molecular markers in the blood of healthy volunteers and patients with PBC at different stages of the disease were studied. Initially, the distribution of the patients according to the stages of the disease was made based on the values of generally accepted markers, such as ALT, AST6 GGT and ALP, as well as the results of the clinical examination. Then, venous blood samples were taken from the patients at different stages of PBC, and the values of the TNF-α , IL-6, Nf-kB, MCP-1/CCL2, Bcl-2, and MDA molecular markers were determined in these samples. Thus, the concentration of the molecular markers in different stages of PBC were determined.

Marker concentration values are given as a percentage, wherein:
0% - the marker is undefinable;
100% - the maximum concentration value of the marker for PBC.

Stage 1 of PBC is a trigger that initiates damage to liver cells. Reference marker concentration values are following:
1) IL-6 - 9.9-10.1%;
2) Nf-kB - 4.5-5.5%;
3) MCP-1/CCL2-4.5-5.5%;
4) Bcl-2 - 4.5-5.5%;
5) TNF-α - 10.8-13.2%;
6) MDA - 0-3.3%.

Stage 2 of PBC - the development of cholestasis and the induction of inflammation. Reference marker concentration values are following:
1) IL-6-27-33%;
2) Nf-kB - 31.5-38.5%;
3) MCP-1/CCL2 - 33.3-40.7%;
4) Bcl-2 - 18-22%;
5) TNF-α - 27.9-34.1 %;
6) MDA - 4.5-5.5%.

Stage 3 of PBC - the development of high intensity inflammation. Reference marker concentration values are following:
1) IL-6-72-88%;
2) Nf-kB - 67.5-82.5%;
3) MCP-1/CCL2 - 7 -88%;
4) Bcl-2 - 36-44%;
5) TNF-α - 58.5-71.5%;
6) MDA - 22.5-27.5%.

Stage 4 of PBC - fibrosis. Reference marker concentration values are following:
1) IL-6-72-88%;
2) Nf-kB - 67.5-82.5%;
3) MCP-1/CCL2 - 72-88%;
4) Bcl-2 - 72-88%;
5) TNF-α - 72.9-89.1 %;
6) MDA - 40.5-49.5%.

Stage 5 of PBC - cirrhosis. Reference marker concentration values are following:
1) IL-6 - 36-44%;
2) Nf-kB - 40.5-49.5%;
3) MCP-1/CCL2 - 31.5-38.5%;
4) Bcl-2 - 36-44%;
5) TNF-α - 45.9-56.1%;
6) MDA - 58.5-71.5%.

As can be seen from the scheme above, determining concentration values of the claimed group of the markers enables understanding types and intensity of the pathological processes occurring in the liver and enables clearly differentiating the stages of PBC and adequately prescribing or adjusting the treatment. The claimed markers enable determining PBC already from the first stage of the disease. In addition, the claimed markers demonstrate the occurrence of the inflammation and apoptosis processes already from the initial stages of PBC, thus proving the need for the use of OCA in combination with UDCA starting from the first stages of the disease.

Moreover, the use of the claimed group of the markers enables diagnosing PBC in the early stage. For example, when the patient has been diagnosed with AMA and ANA antibodies, but liver tests are adequate, the physician will most likely not diagnose PBC. As a result, a dangerous situation arises when, without proper treatment, the disease will progress to further stages. At the same time, the study of the claimed group of the markers is more accurate because it will show elevated concentration values, despite relatively adequate liver tests, which determines prescription of the drugs.

### PBC TREATMENT

The development of PBC is associated with the loss of the immune tolerance to PDC-E2. Defective clearance of apoptotic bodies that are released during the death of the biliary endothelial cells (BECs) is a possible source of autoantigens and further autoimmune reactions. Thus, intact, and antigenically reactive PDC-E2, that was found on the membrane surface of the apoptotic bodies from cultured human cholangiocytes, exhibits on the apotope surface that is absent in other cell types. These apotopes can stimulate the immune response and are available for AMA, which explains the occurrence of the selective autoimmunity against the bile ducts in PBC.

In addition, the apoptotic bodies released from cholangiocytes, in combination with AMA, stimulate a surge in concentration values of pro-inflammatory cytokines.

In addition to PDC-E2, other mitochondrial autoantigens, including the E2 subunit of the oxoglutarate dehydrogenase complex and the E2 subunit of the branched-chain 2-oxoacid dehydrogenase complex, were also present in the apoptotic bodies that were released from cholangiocytes exposed to the toxic bile acids, and thus an autoimmune reaction occurs.

During the development of the disease, degrading cells (hepatocytes and cholangiocytes) release mediators that affect sinusoidal cells, among which are stellate cells - the main cells that synthesize the matrix in the damaged liver. In the normal condition, stellate cells are located in the Disse space, and they are the main depot for vitamin A and are involved in the regulation of fibrogenesis. As a result of the progressive chronic injury, stellate cells become activated and differentiate into myofibroblast-like cells, acquiring contractile, proinflammatory, and profibrotic properties. In the future, this leads to a disruption in the interaction of fibrous and antifibrotic mechanisms, which leads to excessive synthesis of the extracellular matrix and the formation of liver fibrosis.

In particular, Kupffer cells, regional macrophages of the liver, are important effector cells for the inflammation development. When liver cell death starts as a result of the autoimmune processes, it is Kupffer cells that react to damage-associated molecular fragments that are released from dead cells (DAMP) and stimulate further inflammation. It is they that recruit circulating blood monocytes to the liver, and stimulate the stellate cells of the liver, which begin to produce connective tissue, which leads to fibrosis. This, in turn, leads to the further development of the inflammation, fibrosis, cirrhosis and liver cancer.

As mentioned above, currently for the PBC treatment, pharmaceutical compositions comprising ursodeoxycholic acid are used first, and, in the absence of the effectiveness or in the case of intolerance, pharmaceutical compositions with obeticholic acid are prescribed. In order to better understand the disadvantages of this approach, it is necessary to consider the mechanisms of action of both acids.

Experimental data indicate three main mechanisms of action of UDCA:
- protection of cholangiocytes against cytotoxic hydrophobic bile acids;
- stimulation of hepatobiliary secretion;
- protection of hepatocytes from apoptosis caused by bile acids.

One of the protective mechanisms of UDCA against cholestasis is the utilization of the accumulated bile acids. However, the secretory ability of hepatocytes is closely related to the presence of the transport proteins in the tubular membrane. In this regard, UDCA stimulates the expression of the bile salt export pump proteins (BSEP), various drug resistance-associated proteins (MDR3 and MRP4), and promotes the elimination of the bile acids from hepatocytes.

In addition, another mechanism of protection of hepatocytes against the toxic bile acids is the inhibition of FA uptake by the transporters in the intestinal lumen due to the competition of UDCA for binding sites with the transporters.

Clinical studies on the effectiveness of UDCA have demonstrated a decrease in concentration values of aminotransferases, alkaline phosphatase, and bilirubin in the patient blood serum, regardless of improvements in the processes at the histological level. Discussions remain regarding the effectiveness of UDCA in terms of overall patient survival, the impact on the need for liver transplantation, or the achievement of improvements at the histological level.

The debate about whether UDCA affects survival and mortality in the patients without liver transplantation is highlighted in review articles and meta-analyses, where scientists agree on some control of the biochemical markers in the patients taking UDCA, while the impact on histopathology and survival without transplantation cannot be assessed during study. However, the fact is that UDCA is not an ideal drug for the PBC treatment, and it does not always stop the disease progression.

In addition, asymptomatic cases of PBC have a favorable natural history and cannot be considered in the same cohort as symptomatic PBC. Of particular note, two out of three asymptomatic PBC patients benefit from the use of UDCA, compared with an equal or better history with reports of 57%, 70%, and over 90% of 10-year survival in the patients who received no treatment. Additionally, the expected median survival of the asymptomatic PBC patients was 10 and 16 years in two large cohorts observed for 24 years, while the median survival of the symptomatic patients was approximately 7 years.

A systematic review of 16 randomized clinical trials evaluating the efficacy of UDCA versus placebo in PBC showed no significant effect of UDCA on mortality or the need for liver transplantation in the patients. The effects of UDCA do not go beyond a certain improvement in the bilirubin level in the blood serum, liver tests of ALT and AST, and alkaline phosphatase. UDCA has not been found to improve the patient symptoms such as itching and fatigue, autoimmune conditions, liver histology, and portal pressure. In addition, the use of UDCA was associated to significant extend with the development of adverse events, such as weight gain.

Given these data, it can be concluded that, although UDCA has some therapeutic activity and proven efficacy, it is effective only in the early stages of PBC and in the asymptomatic patients.

Mechanism of action of obeticholic acid. OCA is a derivative of the primary human bile acid, chenodeoxycholic acid, and is the first agonist in its class to selectively bind to the nuclear farnesoid X receptor FXR. Due to chemical modification, the affinity of OCA to FXR is 100 times higher than that of chenodeoxycholic acid that is a natural FXR agonist. FXR is one of the key transcription factors that is overexpressed in the liver, kidneys, intestines, and adrenal glands and plays a key role in the pathogenesis of the inflammatory liver diseases. FXR has anti-inflammatory properties due to inhibition of the NF-_{K}B gene. FXR regulates bile acid synthesis through two distinct mechanisms. In the liver, FXR increases the expression of the SHP (small heterodimer partner) gene. The SHP gene represses the transcription of the bile acid synthesis enzymes, such as CYP7A1 and CYP8B1, thus reducing the bile acid synthesis in hepatocytes. In addition, FXR regulates the activity of the bile acid transport systems in hepatocytes. In hepatocytes, FXR is involved in liver inflammation, fibrosis, regulation of metabolic pathways, and regeneration.

In addition to its central role in the bile acid metabolism, FXR activation also regulates the expression of various genes involved in glucose, lipid, and lipoprotein metabolism. Hepatic FXR inhibits fatty acid synthesis and absorption and enhances beta-oxidation by regulating lipid homeostasis.

OCA suppresses metabolic stress-induced activation of the p53 protein and apoptosis of damaged hepatocytes. Moreover, in addition to hepatocytes, OCA has been shown to exhibit anti-inflammatory and antifibrotic properties in immune cells, vascular smooth muscle cells, endothelial cells, and fibroblasts in vitro and in vivo.

It was also shown that stimulation of FXR in hepatic macrophage cells by OCA had an anti-inflammatory effect, inhibited the development of fibrosis, and stimulated liver tissue regeneration.

Comparison of the action of UDCA and OCA. As mentioned above, the main mechanisms of action of UDCA are immunomodulatory action, inhibition of absorption of hydrophobic FAs from the intestinal lumen and stimulation of FA release from hepatocytes, while for OCA these are modulation of FXR action, anti-inflammatory and antifibrotic effects, and inhibition of FA synthesis.

Based on the above scheme of 5 stages in the development of PBC (1 - a trigger that initiates damage to liver cells; 2 - development of cholestasis and induction of inflammation; 3 - development of high-intensity inflammation; 4 - fibrosis; 5 - cirrhosis), today, UDCA is used in the first two stages, since its anti-inflammatory properties are extremely weak, and OCA is used from the second to the last stage. Starting from the second stage of PBC, it is no longer rational to waste time treating the patient with UDCA pharmaceutical composition, since the percentage of the development and transition to irreversible stages of the disease is high. Therefore, an accurate and timely diagnosis of the PBC stage will lead to the possibility of prescribing safer and more effective treatment and use of a mixture/composition of two acids.

### EMBODIMENTS OF THE INVENTION

To explore the possibility of using new markers for the PBC diagnosis, molecular and biological studies of the concentration values of the markers of inflammation, apoptosis, and lipid peroxidation were carried out in the patients diagnosed with PBC and healthy volunteers. The results are listed below in Tables 1-3.

**Table 1**

| Anthropomorphic data of healthy volunteers and patients diagnosed with PBC | | |
|---|---|---|
| | Healthy volunteers (N=58) | Patients with PBC (N=60) |
| Age (years) | | |
| Mean value (years) | 59.24 | 59.6 |
| Range (years) | 41-78 | 47-79 |
| Sex | | |
| Men (N) | 10 | 5 |
| Women (N) | 48 | 55 |
| Laboratory markers (mean value) | | |
| ALT (U/I) | 19.5 | 83 |
| AST (U/I) | 25.94 | 110 |
| GGT (U/I) | 23.4 | 210.5 |
| AP (U/I) | 87 | 354 |
| Total bilirubin (µmol) | 9.2 | 44.2 |
| Presence of AMA antibodies (%) | 0 | 98 |
| IgM (g/l) | 0.6 | 5.21 |

To determine the concentration values of the markers in the blood serum, chemiluminescence and enzyme immunoassay methods were used.

**Table 2**

| The concentration values of inflammatory and apoptotic markers in the blood serum of healthy volunteers and patients diagnosed with PBC | | |
|---|---|---|
| | Healthy volunteers | Patients with PBC |
| TNF-α (ng/ml) | 0.125 | 0.2975 |
| IL-6 (pg/ml) | 2.15 | 17.95 |
| Nf-kB (pg/ml) | 12.2 | 24.78 |
| MCP-1/CCL2 (pg/ml) | 212.2 | 375.9 |
| Bcl-2 (U/ml) | 0.22 | 0.33 |
| MDA (nmol/ml) | 4.21 | 16.98 |

Table 3 shows structured data about concentration values of markers, which were identified in different stages of PBC.

**Table 3**

| The concentration level values of inflammatory and apoptotic markers in the blood serum of healthy volunteers and patients by stages of PBC | | | | | |
|---|---|---|---|---|---|
| | Healthy volunteers | 1 st stage | 2nd stage | 3rd stage | 4th stage |
| TNF-a (ng/ml) | 0.125 | 0.18 | 0.25 | 0.34 | 0.42 |
| IL-6 (pg/ml) | 2.15 | 6.2 | 12.5 | 24.5 | 28.6 |
| Nf-kB (pg/ml) | 12.2 | 15.5 | 20.8 | 28.5 | 34.3 |
| MCP-1/CCL2 (pg/ml) | 212.2 | 250.5 | 311.2 | 457.3 | 484.6 |
| Bcl-2 (U/ml) | 0.22 | 0.031 | 0.4 | 0.48 | 0.41 |
| MDA (nmol/ml) | 4.2 | 8.5 | 15.3 | 20.1 | 24.02 |

Based on the results obtained, it can be concluded that in the patients with PBC, there were found increased concentration values of the markers of inflammation (TNF-α, IL-6, Nf-kB, and MCP-1), apoptosis (Bcl-2), as well as lipid peroxidation marker (MDA), in the blood.

### Results interpretation

TNF-α, IL-6, Nf-kB, MCP-1 are powerful markers of inflammation in tissues. Their increased activity indicates the acute phase of inflammation. In order to inhibit this inflammation, it is necessary to use strong anti-inflammatory drugs with local action. That is, the manifestation of such markers enables using not only UDCA, but switching immediately to the mixture of acids, such as UDCA and OCA.

Bcl-2 is mitochondrial apoptosis factor. The elevated concentration values indicate pathological cell death. This pathological phenomenon can be eliminated by neutralizing the primary cause of the cell death (in this case, this is the toxic effect of the bile acids and the autoimmune process). Again, another marker that confirms the need to use the mixture: for the autoimmune process - UDCA, and to remove and stop the synthesis of fatty acids - UDCA and OCA together.

MDA is a marker of lipid peroxidation. Its increase indicates a high concentration of highly reactive oxygen in the tissues, which can be caused by both inflammation and the immune response.

In general, determination of the markers claimed by the authors enables achieving the following advantages:
1) First of all, their use enables diagnosing PBC in very early stages, before the destruction of the liver cells and fibrosis begins, while biochemical markers (ALT/AST and bilirubin) show not the onset, but the progression of the disease. Successful diagnosis and early-stage treatment of the disease, in turn, enables bringing rates of adequate response to treatment in patients closer to 100%.
2) Choosing the right treatment strategy. Without understanding the molecular pathogenesis, it is impossible to choose the correct individual treatment.
3) More detailed and accurate tracking of the dynamics of the disease and more effective adjustments of the treatment based on the patient condition.

After obtaining a complete clinical picture of the disease based on the results of the study of the claimed markers, one can proceed directly to treatment.

To date, there is no pharmaceutical composition that is effective for the treatment of PBC in all stages of its course.

As a result of the studies on the use of the claimed markers in the PBC treatment, it was shown that in the patients diagnosed with PBC, there is an increase in the markers of inflammation and apoptosis, which indicates the feasibility of changing the protocols for the PBC treatment, in particular, the need for simultaneous use of UDCA and OCA in one dosage form.

For these purposes, a new all-purpose pharmaceutical composition for the PBC treatment, that comprises both ursodeoxycholic and obeticholic acids, and is effective for the use in all stages of PBC and has a complex mechanism of action, was developed. In particular, the use of the new pharmaceutical composition enables simultaneous blockage of the transport and synthesis of bile acids and achievement of a pronounced hepatoprotective effect.

According to some embodiments of the present disclosure, the claimed pharmaceutical composition may have the following composition:

| | Description | Composition |
|---|---|---|
| Embodiment I | Coated tablet | Ursodeoxycholic acid - 100 mg |
| | | Obeticholic acid - 1 mg |
| | | Excipient |
| Embodiment II | Uncoated tablet | Ursodeoxycholic acid - 100 mg |
| | | Obeticholic acid - 2.5 mg |
| | | Excipient |
| Embodiment III | Capsule | Ursodeoxycholic acid - 100 mg |
| | | Obeticholic acid - 5 mg |
| | | Excipient |
| Embodiment IV | Coated tablet | Ursodeoxycholic acid - 100 mg |
| | | Obeticholic acid - 10 mg |
| | | Excipient |
| Embodiment V | Uncoated tablet | Ursodeoxycholic acid - 100 mg |
| | | Obeticholic acid - 20 mg |
| | | Excipient |
| Embodiment VI | Capsule | Ursodeoxycholic acid - 100 mg |
| | | Obeticholic acid - 50 mg |
| | | Excipient |
| Embodiment VII | Coated tablet | Ursodeoxycholic acid - 250 mg |
| | | Obeticholic acid - 1 mg |
| | | Excipient |
| Embodiment VIII | Uncoated tablet | Ursodeoxycholic acid - 250 mg |
| | | Obeticholic acid - 2.5 mg |
| | | Excipient |
| Embodiment IX | Capsule | Ursodeoxycholic acid - 250 mg |
| | | Obeticholic acid - 5 mg |
| | | Excipient |
| Embodiment X | Coated tablet | Ursodeoxycholic acid - 250 mg |
| | | Obeticholic acid - 10 mg |
| | | Excipient |
| Embodiment XI | Uncoated tablet | Ursodeoxycholic acid - 250 mg |
| | | Obeticholic acid - 20 mg |
| | | Excipient |
| Embodiment XII | Capsule | Ursodeoxycholic acid - 250 mg |
| | | Obeticholic acid - 50 mg |
| | | Excipient |
| Embodiment XIII | Coated tablet | Ursodeoxycholic acid - 500 mg |
| | | Obeticholic acid - 1 mg |
| | | Excipient |
| Embodiment XIV | Uncoated tablet | Ursodeoxycholic acid - 500 mg |
| | | Obeticholic acid - 2.5 mg |
| | | Excipient |
| Embodiment XV | Capsule | Ursodeoxycholic acid - 500 mg |
| | | Obeticholic acid - 5 mg |
| | | Excipient |
| Embodiment XVI | Coated tablet | Ursodeoxycholic acid - 500 mg |
| | | Obeticholic acid - 10 mg |
| | | Excipient |
| Embodiment XVII | Uncoated tablet | Ursodeoxycholic acid - 500 mg |
| | | Obeticholic acid - 20 mg |
| | | Excipient |
| Embodiment XVIII | Capsule | Ursodeoxycholic acid - 500 mg |
| | | Obeticholic acid - 50 mg |
| | | Excipient |
| Embodiment XIX | Coated tablet | Ursodeoxycholic acid - 1000 mg |
| | | Obeticholic acid - 1 mg |
| | | Excipient |
| Embodiment XX | Uncoated tablet | Ursodeoxycholic acid - 1000 mg |
| | | Obeticholic acid - 2.5 mg |
| | | Excipient |
| Embodiment XXI | Capsule | Ursodeoxycholic acid - 1000 mg |
| | | Obeticholic acid - 5 mg |
| | | Excipient |
| Embodiment XXII | Coated tablet | Ursodeoxycholic acid - 1000 mg |
| | | Obeticholic acid - 10 mg |
| | | Excipient |
| Embodiment XXIII | Uncoated tablet | Ursodeoxycholic acid - 1000 mg |
| | | Obeticholic acid - 20 mg |
| | | Excipient |
| Embodiment XXIV | Capsule | Ursodeoxycholic acid - 1000 mg |
| | | Obeticholic acid - 50 mg |
| | | Excipient |

According to preferred embodiments of the present disclosure, the claimed pharmaceutical composition may have the following composition:

| | Description | Composition |
|---|---|---|
| Embodiment XXX | Coated tablet | Ursodeoxycholic acid - 150 mg |
| | | Obeticholic acid - 1 mg |
| | | Excipient |
| Embodiment XXXI | Uncoated tablet | Ursodeoxycholic acid - 250 mg |
| | | Obeticholic acid - 1 mg |
| | | Excipient |
| Embodiment XXXII | Capsule | Ursodeoxycholic acid - 250 mg |
| | | Obeticholic acid - 1.25 mg |
| | | Excipient |
| Embodiment XXXIII | Coated tablet | Ursodeoxycholic acid - 150 mg |
| | | Obeticholic acid - 2 mg |
| | | Excipient |
| Embodiment XXXIV | Uncoated tablet | Ursodeoxycholic acid - 250 mg |
| | | Obeticholic acid - 2.5 mg |
| | | Excipient |
| Embodiment XXXV | Capsule | Ursodeoxycholic acid - 500 mg |
| | | Obeticholic acid - 2.5 mg |
| | | Excipient |
| Embodiment XXXVI | Coated tablet | Ursodeoxycholic acid - 750 mg |
| | | Obeticholic acid - 2.5 mg |
| | | Excipient |
| Embodiment XXXVII | Uncoated tablet | Ursodeoxycholic acid - 150 mg |
| | | Obeticholic acid - 5 mg |
| | | Excipient |
| Embodiment XXXVIII | Capsule | Ursodeoxycholic acid - 250 mg |
| | | Obeticholic acid - 5 mg |
| | | Excipient |
| Embodiment XXXIX | Coated tablet | Ursodeoxycholic acid - 500 mg |
| | | Obeticholic acid - 5 mg |
| | | Excipient |
| Embodiment XXXVII | Uncoated tablet | Ursodeoxycholic acid - 750 mg |
| | | Obeticholic acid - 5 mg |
| | | Excipient |
| Embodiment XL | Capsule | Ursodeoxycholic acid - 150 mg |
| | | Obeticholic acid - 10 mg |
| | | Excipient |
| Embodiment XLI | Coated tablet | Ursodeoxycholic acid - 250 mg |
| | | Obeticholic acid - 10 mg |
| | | Excipient |
| Embodiment XLI | Uncoated tablet | Ursodeoxycholic acid - 500 mg |
| | | Obeticholic acid - 10 mg |
| | | Excipient |
| Embodiment XLII | Capsule | Ursodeoxycholic acid - 750 mg |
| | | Obeticholic acid - 10 mg |
| | | Excipient |

Above-mentioned embodiments can be produced, for example, in the following ways.

### Method 1.

Step 1: Weighing. Weigh the required amount of the active and auxiliary components.

Step 2: Sifting of the raw materials. Sift the raw materials through a sieve with a specific mesh size, in accordance with the technical instructions for the manufacture of the solid dosage form.

Step 3: Dry mixing. Transfer the sifted materials to a dry mixing apparatus and mix for a specific amount of time at a specific mixing speed in accordance with the technical instructions for the manufacture of the solid dosage form to produce mixed powder.

Step 4: Producing a slurry. Mix a specific volume of the purified water EP having a specific temperature with a base to produce a slurry.

Step 5: Blending and bonding. Granulation. Mix the slurry produced in Step 4 with the powder from Step 3 in the granulator.

Step 6: Semi-drying. Transfer the granules produced in Step 5 to the drying apparatus and dry at a specific temperature for a specific amount of time, in accordance with the technical instructions for the manufacture of the solid dosage form to produce dried granules.

Step 7: Crushing and sifting all dried granules from Step 6 to produce sifted granules.

Step 8: Final drying. Transfer the sifted granules produced in Step 7 to the drying apparatus and dry at a specific temperature for a specific amount of time, in accordance with the technical instructions for the manufacture of the solid dosage form.

Step 9: Lubricating.

Transfer all granules produced in Step 8 to a blender and mix for a specific amount of time. Add a material to lubricate the granules and mix for a specific amount of time.

Step 10: Tablet compression.

Step 11: Tablet coating.

### Method 2.

Step 1: Weighing. Weigh the required amount of the active and auxiliary components.

Step 2: Sifting of the raw materials. Sift the raw materials through a sieve with a specific mesh size, in accordance with the technical instructions for the manufacture of the solid dosage form.

Step 3: Dry mixing. Transfer the sifted materials to a dry mixing apparatus and mix for a specific amount of time at a specific mixing speed in accordance with the technical instructions for the manufacture of the solid dosage form to produce mixed powder.

Step 4: Adding granulating solution and wetting.

Add pre-prepared granulating solution to the mixed powder produced in Step 3 and mix to produce mixture.

Step 5: Transfer the mixture produced in Step 4 to the granulator and mix to produce granules.

Step 6: Drying. Transfer the granules produced in Step 5 to the drying apparatus and dry at a specific temperature for a specific amount of time, in accordance with the technical instructions for the manufacture of the solid dosage form.

Step 7: Granule coating.

### Method 3.

Step 1: Weighing. Weigh the required amount of the active and auxiliary components.

Step 2: Sifting of the raw materials. Sift the raw materials through a sieve with a specific mesh size, in accordance with the technical instructions for the manufacture of the solid dosage form.

Step 3: Dry mixing. Transfer the sifted materials to a dry mixing apparatus and mix for a specific amount of time at a specific mixing speed in accordance with the technical instructions for the manufacture of the solid dosage form to produce mixed powder.

Step 4: Preparing of the granulating solution. Add the granulating solution base and purified water EP into the preparation container and mix for a specific amount of time in accordance with the technical instructions for the manufacture of the solid dosage form to produce granulating solution.

Step 5: Adding of the granulating solution and wetting.

To the dry mixture produced in Step 3, add the granulating solution produced in Step 4 and mix to produce mixture.

Step 6: Transfer the mixture produced in Step 5 to the granulator and mix to produce granules.

Step 7: Drying. Transfer the granules produced in Step 6 to the drying apparatus and dry at a specific temperature for a specific amount of time, in accordance with the technical instructions for the manufacture of the solid dosage form.

Step 8: Granule coating.

### Method 4.

Step 1: Weighing. Weigh the required amount of the active and auxiliary components.

Step 2: Sifting of the raw materials. Sift the raw materials through a sieve with a specific mesh size, in accordance with the technical instructions for the manufacture of the solid dosage form.

Step 3: Dry mixing. Transfer the sifted materials to a dry mixing apparatus and mix for a specific amount of time at a specific mixing speed in accordance with the technical instructions for the manufacture of the solid dosage form to produce mixed powder.

Step 4: Tablet compression.

Step 5: Tablet coating.

As an excipient, claimed pharmaceutical composition can comprise any excipients acceptable for use in pharmaceutical industry, in particular, excipients that are used to produce solid dosage forms, such as fillers, binders, antiadherents, glidants, lubricants, disintegrants, plasticizer, sweeteners, opaquants, flavorants.

As used herein, the term "filler" is intended to mean inert substances used to create the desired bulk, flow properties, and compression characteristics in the preparation of tablets and capsules. Exemplary fillers include, but not limited to, dibasic calcium phosphate, kaolin, lactose, sucrose, mannitol, cellulose and its derivatives, precipitated calcium carbonate, sorbitol, and starch.

As used herein, the term "binder" is intended to mean a substance used to cause adhesion of powder particles in granulations. Exemplary binders include, but not limited to, acacia, tragacanth, alginic acid and salts thereof, gelatin, cellulose and its derivatives, poly(vinylpyrrolidone), liquid glucose, povidone, polyethylene glycol, polypropylene glycol, polyoxyethylene-polypropylene copolymer, polyethylene ester, polyethylene sorbitan ester, polyethylene oxide, guar gum, polysaccharide, bentonites, sugars, invert sugars, compressible sugar, poloxamers, collagen, albumin, and starch and its derivatives.

As used herein, the term "antiadherent" is intended to mean an agent that prevents the sticking of tablet formulation ingredients to punches and dies in a tableting machine during production. Exemplary antiadherents include, but not limited to, magnesium stearate, talc, calcium stearate, glyceryl behenate, polyethylene glycol (PEG), hydrogenated vegetable oil, mineral oil, stearic acid.

As used herein, the term "glidant" is intended to mean an agent used in tablet and capsule formulations to promote flowability of the granulation. Exemplary glidants include, but not limited to, colloidal silica, cornstarch, talc, calcium silicate, magnesium silicate, colloidal silicon, silicon hydrogel.

As used herein, the term "lubricant" is intended to mean a substance used to reduce friction during compression or other processing. Exemplary lubricants include, but not limited to, calcium stearate, magnesium stearate, mineral oil, stearic acid, and zinc stearate.

As used herein, the term "disintegrant" is intended to mean a compound used to promote the disruption of the solid mass into smaller particles that are more readily dispersed or dissolved. Exemplary disintegrants include, but not limited to, starches such as corn starch, potato starch, pregelatinized and modified starches thereof, clays, such as bentonite, cellulose and its derivatives, such as microcrystalline cellulose, carboxymethylcellulose calcium, cellulose polyacrilin potassium, alginates, sodium starch glycolate, gums such as agar, guar, locust bean, karaya, pectin, tragacanth; crospovidone.

As used herein, the term "plasticizer" includes compounds capable of plasticizing or softening a polymer or binder used in invention by way of lowering the melting temperature or glass transition temperature of the polymer or binder. Exemplary plasticizers include, but not limited to, low molecular weight polymers, oligomers, copolymers, oils, low molecular weight polyols, multi-block polymers, single block polymers, and glycerin.

As used herein, the term "sweetener" is intended to mean a compound used to impart sweetness. Exemplary sweeteners include, but not limited to, aspartame, dextrose, glycerin, mannitol, saccharin sodium, sorbitol and sucrose.

As used herein, the term "opaquant" is intended to mean a compound used to render a tablet coating opaque. Exemplary opaquant include, but not limited to, titanium dioxide, talc.

As used herein, the term "flavorant" is intended to mean a compound used to impart a pleasant flavor and often odor. Exemplary flavorants include, but not limited to, synthetic flavor oils and flavoring aromatics and/or natural oils, extracts from plants, leaves, flowers, fruits and combinations thereof, and other substances known to one skilled in the art.

### Method A.

Step 1. Weighing. Weigh the required amount of the active and auxiliary components (per 100 kg of the total mixture).
1) UDCA + OCA - 80 kg.
2) Corn starch - 15 kg.
3) Lactose - 3 kg.
4) Povidone - 2 kg.

Step 2. Sifting of the raw materials. Sift the raw materials through a sieve with a mesh size of at least 30 mesh.

Step 3. Dry mixing. Transfer the sieved materials to a dry mixer and mix them for 1 hour.

Step 4. Transfer the mixture produced in Step 3 to a compression machine, and start the process of producing the tablets by direct compression.

Step 5. Transfer the tablets produced in Step 4 to a tablet film coating machine. Coating of the tablets with the film achieved by immersion in an ethanol solution of a film former (eudragit E).

Step 6. Drying of the tablets produced.

### Method B.

Step 1. Weighing. Weigh the required amount of the active and auxiliary components (per 150 kg of the total mixture).
1) UDCA + OCA - 112.5 kg.
2) Corn starch - 16.5 kg.
3) Lactose - 18.0 kg.
4) Povidone - 3.0 kg.

Step 2. Sifting of the raw materials. Sift the raw materials through a sieve with a mesh size of at least 30 mesh.

Step 3. Dry mixing. Transfer the sieved materials to a dry mixer and mix them for 1 hour.

Step 4: Transfer the mixture produced in Step 3 to a compression machine, and start the process of producing the tablets by direct compression.

Step 5. Transfer the tablets produced in Step 4 to a tablet film coating machine. Coating of the tablets with the film achieved by immersion in an ethanol solution of a film former (eudragit L).

Step 6. Drying of the tablets produced.

### Method C

Step 1. Weighing. Weigh the required amount of the active and auxiliary components (per 200 kg of the total mixture).
1) UDCA + OCA -150 kg.
2) Microcrystalline cellulose - 30 kg.
3) Lactose - 18 kg.
4) Povidone - 2 kg.

Step 2. Sifting of the raw materials. Sift the raw materials through a sieve with a mesh size of at least 30 mesh.

Step 3. Dry mixing. Transfer the sifted materials to a dry mixer and mix them for 1 hour.

Step 4: Transfer the mixture produced in Step 3 to a compression machine, and start the process of producing the tablets by direct compression.

Step 5. Transfer the tablets produced in Step 4 to a tablet film coating machine. Coating of the tablets with the film achieved by immersion in an ethanol solution of a film former (eudragit L).

Step 6. Drying of the tablets produced.

### CLINICAL STUDIES

A double-blind clinical study on the effectiveness of the combined pharmaceutical composition comprising UDCA and OCA compared with the monocomposition comprising only UDCA was conducted for the patients with the following concentration values of the claimed group of the markers (corresponding to 1 Stage of PBC):
- IL-6 - at least 10 %;
- Nf-kB - at least 5 %;
- MCP-1/CCL2 - at least 5 %;
- Bcl-2 - at least 5 %;
- TNF-α - at least 12 %;
- MDA - at least 0.5 %.

**Table 4**

| Data on the tested groups before the treatment | | | |
|---|---|---|---|
| | Group "UDCA 15 mg/kg" (N=68) | Group "UDCA 15 mg/kg + OCA 5 mg" (N=65) | Group "UDCA 15 mg/kg + OCA 10 mg" (N=63) |
| Age (years) | | | |
| Mean value (years) | 59.6 | 58.2 | 59.1 |
| Range (years) | 47-79 | 47-78 | 48-77 |
| Sex | | | |
| Men (N) | 9 | 7 | 3 |
| Women (N) | 59 | 58 | 60 |
| Laboratory markers (mean concentration level values) | | | |
| ALT (U/I) | 82.7 | 71.33 | 84.3 |
| AST (U/I) | 105.2 | 106.9 | 112.5 |
| GGT (U/I) | 207.5 | 212.3 | 208.8 |
| AP (U/I) | 368 | 349 | 325 |
| Total bilirubin (µmol/l) | 40.3 | 38.6 | 41.33 |
| Presence of AMA antibodies (%) | 95 | 98 | 94 |
| IgM (g/l) | 0.5 | 0.7 | 0.58 |
| TNF-α (ng/ml) | 0.33 | 0.35 | 0.33 |
| IL-6 (pg/ml) | 23.54 | 24.87 | 26.2 |
| Nf-kB (pg/ml) | 65.5 | 67.6 | 69.5 |
| MCP-1/CCL2 (pg/ml) | 468.5 | 485.4 | 495.2 |
| Bcl-2 (U/ml) | 0.72 | 0.69 | 0.71 |
| MDA (nmol/ml) | 23.08 | 22.58 | 24.01 |

Comparison of the effectiveness of the combined (UDCA + OCA) pharmaceutical composition and monocomposition (UDCA) was carried out for 6 and 12 months. The patients diagnosed with PBC were divided into 3 groups depending on the treatment regimen:
1) Group UDCA 15 mg/kg;
2) Group UDCA 15 mg/kg + OCA 5 mg;
3) Group UDCA 15 mg/kg + OCA 10 mg.

After the treatment, venous blood samples were taken from the patients and the concentrations of the biochemical and molecular markers were determined.

As markers of the treatment effectiveness, two groups of the markers were used: biochemical and molecular biological.

The first group included: ALT, AST, GGT (Gamma-glutamyl Transferase), AP and total bilirubin.

The second group included: TNF-α, IL-6, Nf-kB, MCP-1/CCL2, BCL-2, and MDA.

The study results are shown in Tables 5-6.

**Table 5**

| Concentration values of the markers in the blood plasma (6 months) | | | | | | |
|---|---|---|---|---|---|---|
| | UDCA 15 mg/kg (N=68) | | UDCA 15 mg/kg + OCA 5 mg (N=65) | | UDCA 15 mg/kg + OCA 10 mg (N=63) | |
| | Before | After | Before | After | Before | After |
| ALT (U/I) | 55.7 | 37.5 | 56.33 | 28.2 | 58.3 | 25.3 |
| AST (U/I) | 58.6 | 39.4 | 59.5 | 31.7 | 61.4 | 25.1 |
| GGT (U/I) | 207.5 | 135.2 | 212.3 | 74.2 | 208.8 | 60.2 |
| AP (U/I) | 297.2 | 190 | 284.5 | 147.2 | 281.2 | 120.2 |
| Total bilirubin (µmol/l) | 57.6 | 20 | 59.8 | 12.8 | 54.3 | 12.1 |
| TNF-α (ng/ml) | 0.33 | 0.29 | 0.35 | 0.123 | 0.33 | 0.1 |
| IL-6 (pg/ml) | 23.54 | 20.2 | 24.87 | 5.15 | 26.2 | 3.15 |
| Nf-kB (pg/ml) | 65.5 | 60.2 | 67.6 | 15.2 | 69.5 | 12.3 |
| MCP-1/CCL2 (pg/ml) | 468.5 | 412.3 | 485.4 | 212.8 | 495.2 | 200.2 |
| Bcl-2 (U/ml) | 0.42 | 0.4 | 0.39 | 0.3 | 0.41 | 0.28 |
| MDA (nmol/ml) | 23.08 | 22.01 | 22.58 | 10.17 | 24.01 | 7.2 |

**Table 6**

| Changes in the marker concentration in the blood plasma (6 months), in % of the initial value | | | | | | |
|---|---|---|---|---|---|---|
| | UDCA 15 mg/kg | | UDCA 15 mg/kg + OCA 5 mg | | UDCA 15 mg/kg + OCA 10 mg | |
| | (N=68) | | (N=65) | | (N=63) | |
| | Before | After | Before | After | Before | After |
| ALT | 100 | 67.32 | 100 | 50.06 | 100 | 43.40 |
| AST | 100 | 67.24 | 100 | 53.28 | 100 | 40.88 |
| GGT | 100 | 65.16 | 100 | 34.95 | 100 | 28.83 |
| AP | 100 | 63.93 | 100 | 51.74 | 100 | 42.75 |
| Total bilirubin | 100 | 34.72 | 100 | 21.40 | 100 | 22.28 |
| TNF-α | 100 | 87.88 | 100 | 35.14 | 100 | 30.30 |
| IL-6 | 100 | 85.81 | 100 | 20.71 | 100 | 12.02 |
| Nf-kB | 100 | 91.91 | 100 | 22.49 | 100 | 17.70 |
| MCP-1/CCL2 | 100 | 88.00 | 100 | 43.84 | 100 | 40.43 |
| Bcl-2 | 100 | 95.24 | 100 | 76.92 | 100 | 68.29 |
| MDA | 100 | 95.36 | 100 | 45.04 | 100 | 29.99 |

Results of the study on the effectiveness of the treatment of the patients with UDCA or UDCA + OCA (12 months) are shown in the Tables below.

**Table 7**

| Concentration values of the markers in the blood plasma (12 months) | | | | | | |
|---|---|---|---|---|---|---|
| | UDCA 15 mg/kg | | UDCA 15 mg/kg + OCA 5 mg | | UDCA 15 mg/kg + OCA 10 mg | |
| | (N=68) | | (N=65) | | (N=63) | |
| | Before | After | Before | After | Before | After |
| ALT (U/I) | 82.7 | 55.67 | 71.33 | 35.7 | 84.3 | 36.59 |
| AST (U/I) | 105.2 | 70.73 | 106.9 | 56.96 | 112.5 | 45.99 |
| GGT (U/I) | 207.5 | 135.2 | 212.3 | 74.2 | 208.8 | 60.2 |
| AP (U/I) | 368.4 | 235.36 | 349 | 180.57 | 325 | 138.93 |
| Total bilirubin (µmol/l) | 40.3 | 26.03 | 38.6 | 14.74 | 41.33 | 9.2 |
| TNF-α (ng/ml) | 0.33 | 0.29 | 0.35 | 0.123 | 0.33 | 0.1 |
| IL-6 (pg/ml) | 23.54 | 20.2 | 24.87 | 5.15 | 26.2 | 3.15 |
| Nf-kB (pg/ml) | 65.5 | 60.2 | 67.6 | 15.2 | 69.5 | 12.3 |
| MCP-1/CCL2 (pg/ml) | 468.5 | 412.3 | 485.4 | 212.8 | 495.2 | 200.2 |
| Bcl-2 (U/ml) | 0.72 | 0.52 | 0.69 | 0.43 | 0.71 | 0.34 |
| MDA (nmol/ml) | 23.08 | 22.01 | 22.58 | 10.17 | 24.01 | 7.2 |

**Table 8**

| Changes in the marker concentration in the blood plasma (12 months), in % of the initial value | | | | | | |
|---|---|---|---|---|---|---|
| | UDCA 15 mg/kg (N=68) | | UDCA 15 mg/kg + OCA 5 mg (N=65) | | UDCA 15 mg/kg + OCA 10 mg (N=63) | |
| | Before | After | Before | After | Before | After |
| ALT | 100 | 67.32 | 100 | 50.06 | 100 | 43.40 |
| AST | 100 | 67.24 | 100 | 53.28 | 100 | 40.88 |
| GGT | 100 | 65.16 | 100 | 34.95 | 100 | 28.83 |
| AP | 100 | 63.93 | 100 | 51.74 | 100 | 42.75 |
| Total bilirubin | 100 | 64.58 | 100 | 38.2 | 100 | 22.28 |
| TNF-α | 100 | 87.88 | 100 | 35.14 | 100 | 30.30 |
| IL-6 | 100 | 85.81 | 100 | 20.71 | 100 | 12.02 |
| Nf-kB | 100 | 91.91 | 100 | 22.49 | 100 | 17.70 |
| MCP-1/CCL2 | 100 | 88.00 | 100 | 43.84 | 100 | 40.43 |
| Bcl-2 | 100 | 72 | 100 | 62.32 | 100 | 47.89 |
| MDA | 100 | 95.36 | 100 | 45.04 | 100 | 29.99 |

The results obtained enable drawing the following conclusions:
1. Ursodeoxycholic acid monocomposition was more effective in reducing the biochemical but not molecular and biological markers of inflammation and apoptosis.
2. The effect of ursodeoxycholic acid on the markers of inflammation, apoptosis and lipid peroxidation was insignificant.
3. The use of the combined preparations enables reducing not only the biochemical markers, but also coping effectively with an acute inflammatory process and ceasing the cell death and development of fibrosis.

More detailed study results per months are given below in Tables 9-14.

**Table 9**

| Concentration level values of the markers in the blood plasma when using the standard treatment regimen No. 1 (UDCA 15 mg/kg) | | | | | | |
|---|---|---|---|---|---|---|
| | Months | | | | | |
| | 0 | 1 | 3 | 6 | 9 | 12 |
| ALT (U/I) | 82.7 | 80.4 | 74.81 | 68.2 | 62.47 | 55.67 |
| AST (U/I) | 105.2 | 100.7 | 87.3 | 81.4 | 74.5 | 70.73 |
| GGT (U/I) | 207.5 | 198 | 178.8 | 156.2 | 141.5 | 135.2 |
| AP (U/I) | 368.4 | 352.5 | 290.6 | 269.45 | 247.5 | 235.36 |
| Total bilirubin (µmol/l) | 40.3 | 39.8 | 33.8 | 32.8 | 28.03 | 26.03 |
| TNF-α (ng/ml) | 0.33 | 0.33 | 0.324 | 0.315 | 0.3 | 0.29 |
| IL-6 (pg/ml) | 23.54 | 23.35 | 22.95 | 22 | 21.48 | 20.2 |
| Nf-kB (pg/ml) | 65.5 | 64.4 | 64.1 | 62.8 | 61.4 | 60.2 |
| MCP-1/CCL2 (pg/ml) | 468.5 | 465.4 | 448 | 431.4 | 419.7 | 412.3 |
| Bcl-2 (U/ml) | 0.72 | 0.68 | 0.62 | 0.58 | 0.54 | 0.52 |
| MDA (nmol/ml) | 23.08 | 22.87 | 22.54 | 22.37 | 22.12 | 22.01 |

**Table 10**

| Changes in the marker concentration in the blood plasma when using the standard treatment regimen No. 1 (UDCA 15 mg/kg), in % of the initial value | | | | | | |
|---|---|---|---|---|---|---|
| | Months | | | | | |
| | 0 | 1 | 3 | 6 | 9 | 12 |
| ALT | 100.00 | 97.22 | 90.46 | 82.47 | 75.54 | 67.32 |
| AST | 100.00 | 95.72 | 82.98 | 77.38 | 70.82 | 67.23 |
| GGT | 100.00 | 95.42 | 86.17 | 75.28 | 68.19 | 65.16 |
| AP | 100.00 | 97.20 | 88.10 | 74.45 | 69.18 | 63.93 |
| Total bilirubin | 100.00 | 96.18 | 87.50 | 77.60 | 70.49 | 64.58 |
| TNF-α | 100.00 | 100.00 | 98.18 | 95.45 | 90.91 | 87.88 |
| IL-6 | 100.00 | 99.19 | 97.49 | 93.46 | 91.25 | 85.81 |
| Nf-kB | 100.00 | 98.32 | 97.86 | 95.88 | 93.74 | 91.91 |
| MCP-1/CCL2 | 100.00 | 99.34 | 95.62 | 92.08 | 89.58 | 88.00 |
| Bcl-2 | 100.00 | 94.44 | 86.11 | 80.56 | 75.00 | 72.22 |
| MDA | 100.00 | 99.09 | 97.66 | 96.92 | 95.84 | 95.36 |

The above data show the change in the concentration values of the biochemical and molecular markers of the PBC development, when using treatment regimen No. 1. It can be seen that the chosen treatment regimen was the most effective in reducing the markers, such as ALT, AST, GGT, AP, total bilirubin and BCL-2, which indicates that such a treatment regimen is somewhat effective, but it has little effect on inflammation processes.

**Table 11**

| Concentration values of the markers in the blood plasma when using the provided treatment regimen No. 2 (UDCA 15 mg/kg + OCA 5 mg) | | | | | | |
|---|---|---|---|---|---|---|
| | Months | | | | | |
| | 0 | 1 | 3 | 6 | 9 | 12 |
| ALT (U/I) | 71.33 | 69.7 | 61.7 | 52.75 | 41.79 | 35.7 |
| AST (U/I) | 106.9 | 101.4 | 87 | 79.45 | 71.7 | 56.96 |
| GGT (U/I) | 212.3 | 207.4 | 184.4 | 134.6 | 87.5 | 74.2 |
| AP (U/I) | 349 | 332.3 | 285.6 | 245.8 | 210.5 | 180.57 |
| Total bilirubin (µmol/l) | 38.6 | 37.6 | 31.05 | 24.8 | 16.87 | 14.74 |
| TNF-α (ng/ml) | 0.35 | 0.27 | 0.24 | 0.178 | 0.14 | 0.123 |
| IL-6 (pg/ml) | 24.87 | 18.6 | 16.7 | 12.6 | 8.9 | 5.15 |
| Nf-kB (pg/ml) | 67.6 | 59.7 | 41.5 | 29.7 | 19.4 | 15.2 |
| MCP-1/CCL2 (pg/ml) | 485.4 | 403.6 | 301.5 | 270 | 235.4 | 212.8 |
| Bcl-2 (U/ml) | 0.69 | 0.67 | 0.62 | 0.51 | 0.45 | 0.43 |
| MDA (nmol/ml) | 22.58 | 19.68 | 17.8 | 13.8 | 12.6 | 10.17 |

**Table 12**

| Changes in the marker concentration in the blood plasma when using the provided treatment regimen No. 2 (UDCA 15 mg/kg + OCA 5 mg), in % of the initial value | | | | | | |
|---|---|---|---|---|---|---|
| | Months | | | | | |
| | 0 | 1 | 3 | 6 | 9 | 12 |
| ALT | 100.00 | 97.71 | 86.50 | 73.95 | 58.59 | 50.05 |
| AST | 100.00 | 94.86 | 81.38 | 74.32 | 67.07 | 53.28 |
| GGT | 100.00 | 97.69 | 86.86 | 63.40 | 41.22 | 34.95 |
| AP | 100.00 | 95.60 | 84.91 | 70.29 | 57.32 | 51.74 |
| Total bilirubin | 100.00 | 98.16 | 76.76 | 59.87 | 49.16 | 38.19 |
| TNF-α | 100.00 | 77.14 | 68.57 | 50.86 | 40.00 | 35.14 |
| IL-6 | 100.00 | 74.79 | 67.15 | 50.66 | 35.79 | 20.71 |
| Nf-kB | 100.00 | 88.31 | 61.39 | 43.93 | 28.70 | 22.49 |
| MCP-1/CCL2 | 100.00 | 83.15 | 62.11 | 55.62 | 48.50 | 43.84 |
| Bcl-2 | 100.00 | 97.10 | 89.86 | 73.91 | 65.22 | 62.32 |
| MDA | 100.00 | 87.16 | 78.83 | 61.12 | 55.80 | 45.04 |

The above data show the change in the concentration values of the biochemical and molecular markers of the PBC development, when using treatment regimen No. 2. It can be seen that the selected treatment regimen was more effective than regimen No. 1.

In addition to the fact that in this case there was a more significant decrease in the biochemical markers characterizing the general functional condition of the liver, there was also a significant decrease in the markers of inflammation, chemoattraction of monocytes from peripheral blood, apoptosis and lipid peroxidation.

**Table 13**

| Concentration values of the markers in the blood plasma when using the provided treatment regimen No. 3 (UDCA 15 mg/kg + OCA 10 mg) | | | | | | |
|---|---|---|---|---|---|---|
| | Months | | | | | |
| | 0 | 1 | 3 | 6 | 9 | 12 |
| ALT (U/I) | 84.3 | 82.4 | 71.9 | 59.1 | 41.8 | 36.59 |
| AST (U/I) | 112.5 | 109.6 | 85.6 | 69.96 | 51.8 | 45.99 |
| GGT (U/I) | 208.8 | 198.6 | 135 | 106 | 89.5 | 60.2 |
| AP (U/I) | 325 | 319.5 | 289.57 | 210.5 | 178.57 | 138.93 |
| Total bilirubin (µmol/l) | 41.33 | 39.25 | 31.05 | 24.6 | 16.8 | 9.2 |
| TNF-α (ng/ml) | 0.33 | 0.28 | 0.25 | 0.18 | 0.13 | 0.1 |
| IL-6 (pg/ml) | 26.2 | 21.3 | 16.9 | 13.69 | 7.45 | 3.15 |
| Nf-kB (pg/ml) | 69.5 | 57.61 | 35.6 | 22 | 14.6 | 12.3 |
| MCP-1/CCL2 (pg/ml) | 495.2 | 401.7 | 301.8 | 270.2 | 230.8 | 200.2 |
| Bcl-2 (U/ml) | 0.71 | 0.68 | 0.64 | 0.52 | 0.46 | 0.34 |
| MDA (nmol/ml) | 24.01 | 21.5 | 18.6 | 13.8 | 7.92 | 7.2 |

**Table 14**

| Changes in the marker concentration in the blood plasma when using the provided treatment regimen No. 3 (UDCA 15 mg/kg + OCA 10 mg), in % of the initial value | | | | | | |
|---|---|---|---|---|---|---|
| | Months | | | | | |
| | 0 | 1 | 3 | 6 | 9 | 12 |
| ALT | 100.00 | 97.75 | 85.29 | 70.11 | 49.58 | 43.40 |
| AST | 100.00 | 97.42 | 76.09 | 62.19 | 46.04 | 40.88 |
| GGT | 100.00 | 95.11 | 64.66 | 50.77 | 42.86 | 28.83 |
| AP | 100.00 | 98.67 | 74.32 | 62.19 | 47.21 | 42.75 |
| Total bilirubin | 100.00 | 96.50 | 65.56 | 36.10 | 29.10 | 22.28 |
| TNF-α | 100.00 | 84.85 | 75.76 | 54.55 | 39.39 | 30.30 |
| IL-6 | 100.00 | 81.30 | 64.50 | 52.25 | 28.44 | 12.02 |
| Nf-kB | 100.00 | 82.89 | 51.22 | 31.65 | 21.01 | 17.70 |
| MCP-1/CCL2 | 100.00 | 81.12 | 60.95 | 54.56 | 46.61 | 40.43 |
| Bcl-2 | 100.00 | 95.77 | 90.14 | 73.24 | 64.79 | 47.89 |
| MDA | 100.00 | 89.55 | 77.47 | 57.48 | 32.99 | 29.99 |

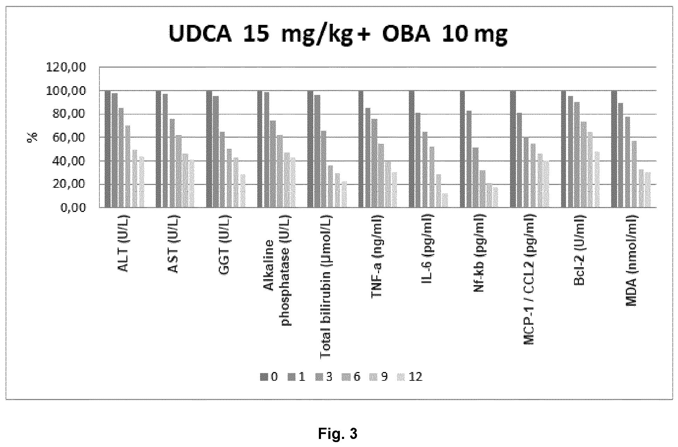

The above data show the change in the concentration values of the biochemical and molecular markers of the PBC development, when using treatment regimen No. 3. Fig. 3 shows that the selected treatment regimen was more effective than regimens No. 1 and No. 2.

When using this regimen, there was a significant decrease in the biochemical markers characterizing the general functional condition of the liver, as well as a significant decrease in the markers of inflammation, chemoattraction of monocytes from peripheral blood, apoptosis and lipid peroxidation.

If we compare all three treatment regimens, we can conclude that:
1) UDCA monocomposition has a specific effect on the overall functional condition of the liver, but almost no effect on the inflammatory and immunological component of the disease.
2) The combined pharmaceutical composition containing UDCA and OCA is more effective in the treatment of PBC, because it affects not only the general functional condition of the liver, but also the molecular mechanisms of the development of this disease, that is, the treatment is not only symptomatic.
3) The use of the new markers enables not only developing the new treatment regimens with the combined pharmaceutical composition, but also evaluating the effectiveness of the treatment in more detail.SOLID DOSAGE FORMS

Specifically, the authors of the present disclosure suggest the manufacture of the claimed pharmaceutical composition comprising obeticholic and ursodeoxycholic acids in the solid dosage form, for example, in the form of a tablet or capsule.

The development of the new all-purpose solid dosage form for the treatment of PBC, that comprises both ursodeoxycholic and obeticholic acids and is characterized by a complex mechanism of action, enables achieving effective treatment in all stages of PBC. In particular, the use of the new solid dosage form enables simultaneous blockage of the transport and synthesis of bile acids, and achievement of a pronounced hepatoprotective effect.

Based on the studies conducted, the authors consider it necessary to use the combined solid dosage form for the treatment of PBC, the active components of which are ursodeoxycholic acid, that stimulates the excretion of bile acids, and obeticholic acid, that stimulates the nuclear FXR receptor, which leads to inhibition of bile acid synthesis, anti-inflammatory, antifibrotic and antiapoptotic effect.

A study of the pharmacokinetics of obeticholic acid in a monocomposition and as part of a composition with ursodeoxycholic acid when taken orally was conducted. It was discovered that when taking OCA with UDCA, UDCA inhibits the absorption of OCA, reducing bioavailability from 89% to 56% for 5 mg OCA, and from 91% to 61% for 10 mg OCA, respectively. The results of the pharmacokinetic studies are shown in Table 15.

**Table 15**

| Pharmacokinetic parameters of the compositions with OBA and UDCA, compared with the solid dosage form according to the present disclosure | | | | | | |
|---|---|---|---|---|---|---|
| | OCA 5 mg | OCA 10 mg | OCA 5 mg + UDCA 500 mg | OCA 10 mg + UDCA 500 mg | OCA 5mg + UDCA 500 mg enteric coating, modified release | OCA 10 mg + UDCA 500 mg enteric coating, modified release |
| Cmax (ng/ml) | 16.7 | 26.4 | 7.1 | 14.8 | 16.2 | 26.8 |
| AUCt (ng*h/ml) | 34.8 | 66.1 | 19.4 | 35.8 | 33.7 | 65.8 |
| Tmax (min) | 40 | 90 | 95 | 140 | 45 | 87 |
| Bioavailability (%) | 89 | 91 | 56 | 61 | 87 | 88 |

Thus, to improve the therapeutic parameters of the combined solid dosage form with ursodeoxycholic and obeticholic acids, the authors of the present disclosure developed the dosage form as a multilayer modified-release tablet. This once again leads to increased compliance of the claimed pharmaceutical composition and solid dosage form and provides additional arguments in favor of the pharmaceutical composition with OCA and UDCA in one dosage form as a tablet with a structural solution. Providing the complexity of the treatment (many different pills) and the specifics of the symptoms with manifestations of fatigue and lack of concentration, the patients, when taking two separate pharmaceutical compositions, will make mistakes in taking medications: both in the number of the tablets and in taking them in time. Moreover, the problem of competition between OCA and UDCA for the transporters was investigated only now by the authors of the present disclosure and was not previously considered in this context. And if the patient takes several different pharmaceutical compositions with a discrete separate content of UDCA and OCA, this will lead to a decrease in the effectiveness of the treatment due to the suppression of OCA by ursodeoxycholic acid.

One of the solutions suggested by the authors is the multilayer tablet, in which the release of OCA occurs first, and only after that the release of UDCA occurs.

According to one of the embodiments of the present disclosure, the tablet consists of the following layers:
1) The outer layer that can be formulated to be enteric (resistant to hydrochloric acid due to special polymers).
2) The second layer comprising obeticholic acid (OCA Layer). According to one of the embodiments, after the dissolution of the outer layer, the release and absorption of OCA occurs in the intestinal lumen.
3) The STOP layer. It consists of a special layer, the swelling and dissolution of which takes about 1-2 hours, which allows the OCA to be completely absorbed. After the dissolution of this layer, the release of UDCA begins.
4) The core comprising ursodeoxycholic acid. After the dissolution of all the above layers, UDCA is released and absorbed. Potentially, this layer could be designed with extended release with a release time of up to 6 hours.

According to another embodiment of the present disclosure, the tablet consists of the following layers:
1) The outer layer, that may be immediate release coating.
2) The second layer comprising obeticholic acid (OCA Layer).
3) The layer that is enteric coating.
4) The core comprising ursodeoxycholic acid, that can be designed with extended release with UDCA release time up to 6 hours.

Some potential embodiments of the claimed solid dosage forms in the tablet form are presented below in Table 16.

**Table 16**

| Embodiments of the claimed solid dosage forms as the tablet | | |
|---|---|---|
| | Description | Composition |
| Embodiment 1 | Coated multilayer tablet with modified release | Core - ursodeoxycholic acid - 100 mg |
| | | STOP-layer |
| | | Layer No. 3 - obeticholic acid - 1 mg |
| | | Outer layer - enteric coating |
| Embodiment 2 | Coated multilayer tablet | Core - ursodeoxycholic acid - 100 mg |
| | with modified release and extended release of UDCA | STOP-layer |
| | | Layer No. 3 - obeticholic acid - 2.5 mg |
| | | Outer layer - film non-enteric coating |
| Embodiment 3 | Coated multilayer tablet with modified release | Core - ursodeoxycholic acid - 100 mg |
| | | STOP-layer |
| | | Layer No. 3 - obeticholic acid - 5 mg |
| | | Outer layer - enteric coating |
| Embodiment 4 | Coated multilayer tablet with modified release and extended release of UDCA | Core - ursodeoxycholic acid - 100 mg |
| | | STOP-layer |
| | | Layer No. 3 - obeticholic acid - 10 mg |
| | | Outer layer - film non-enteric coating |
| Embodiment 5 | Coated multilayer tablet with modified release | Core - ursodeoxycholic acid - 100 mg |
| | | STOP-layer |
| | | Layer No. 3 - obeticholic acid - 20 mg |
| | | Outer layer - enteric coating |
| Embodiment 6 | Coated multilayer tablet with modified release and extended release of UDCA | Core - ursodeoxycholic acid - 100 mg |
| | | STOP-layer |
| | | Layer No. 3 - obeticholic acid - 50 mg |
| | | Outer layer - film non-enteric coating |
| Embodiment 7 | Coated multilayer tablet with modified release | Core - ursodeoxycholic acid - 250 mg |
| | | STOP-layer |
| | | Layer No. 3 - obeticholic acid - 1 mg |
| | | Outer layer - enteric coating |
| Embodiment 8 | Coated multilayer tablet with modified release and extended release of UDCA | Core - ursodeoxycholic acid - 250 mg |
| | | STOP-layer |
| | | Layer No. 3 - obeticholic acid - 2.5 mg |
| | | Outer layer - film non-enteric coating |
| Embodiment 9 | Coated multilayer tablet with modified release | Core - ursodeoxycholic acid - 250 mg |
| | | STOP-layer |
| | | Layer No. 3 - obeticholic acid - 5 mg |
| | | Outer layer - enteric coating |
| Embodiment 10 | Coated multilayer tablet with modified release and extended release of UDCA | Core - ursodeoxycholic acid - 250 mg |
| | | STOP-layer |
| | | Layer No. 3 - obeticholic acid - 10 mg |
| | | Outer layer - film non-enteric coating |
| Embodiment 11 | Coated multilayer tablet with modified release | Core - ursodeoxycholic acid - 250 mg |
| | | STOP-layer |
| | | Layer No. 3 - obeticholic acid - 20 mg |
| | | Outer layer - enteric coating |
| Embodiment 12 | Coated multilayer tablet with modified release and extended release of UDCA | Core - ursodeoxycholic acid - 250 mg |
| | | STOP-layer |
| | | Layer No. 3 - obeticholic acid - 50 mg |
| | | Outer layer - film non-enteric coating |
| Embodiment 13 | Coated multilayer tablet with modified release | Core - ursodeoxycholic acid - 500 mg |
| | | STOP-layer |
| | | Layer No. 3 - obeticholic acid - 1 mg |
| | | Outer layer - enteric coating |
| Embodiment 14 | Coated multilayer tablet with modified release and extended release of UDCA | Core - ursodeoxycholic acid - 500 mg |
| | | STOP-layer |
| | | Layer No. 3 - obeticholic acid - 2.5 mg |
| | | Outer layer - film non-enteric coating |
| Embodiment 15 | Coated multilayer tablet with modified release | Core - ursodeoxycholic acid - 500 mg |
| | | STOP-layer |
| | | Layer No. 3 - obeticholic acid - 5 mg |
| | | Outer layer - enteric coating |
| Embodiment 16 | Coated multilayer tablet with modified release and extended release of UDCA | Core - ursodeoxycholic acid - 500 mg |
| | | STOP-layer |
| | | Layer No. 3 - obeticholic acid - 10 mg |
| | | Outer layer - film non-enteric coating |
| Embodiment 17 | Coated multilayer tablet with modified release | Core - ursodeoxycholic acid - 500 mg |
| | | STOP-layer |
| | | Layer No. 3 - obeticholic acid - 20 mg |
| | | Outer layer - enteric coating |
| Embodiment 18 | Coated multilayer tablet with modified release and extended release of UDCA | Core - ursodeoxycholic acid - 500 mg |
| | | STOP-layer |
| | | Layer No. 3 - obeticholic acid - 5 mg |
| | | Outer layer - film non-enteric coating |
| Embodiment 19 | Coated multilayer tablet with modified release | Core - ursodeoxycholic acid - 1000 mg |
| | | STOP-layer |
| | | Layer No. 3 - obeticholic acid - 1 mg |
| | | Outer layer - enteric coating |
| Embodiment 20 | Coated multilayer tablet with modified release and extended release of UDCA | Core - ursodeoxycholic acid - 1000 mg |
| | | STOP-layer |
| | | Layer No. 3 - obeticholic acid - 2.5 mg |
| | | Outer layer - film non-enteric coating |
| Embodiment 21 | Coated multilayer tablet with modified release | Core - ursodeoxycholic acid - 1000 mg |
| | | STOP-layer |
| | | Layer No. 3 - obeticholic acid - 5 mg |
| | | Outer layer - enteric coating |
| Embodiment 22 | Coated multilayer tablet with modified release and extended release of UDCA | Core - ursodeoxycholic acid - 1000 mg |
| | | STOP-layer |
| | | Layer No. 3 - obeticholic acid - 10 mg |
| | | Outer layer - film non-enteric coating |
| Embodiment 23 | Coated multilayer tablet with modified release | Core - ursodeoxycholic acid - 1000 mg |
| | | STOP-layer |
| | | Layer No. 3 - obeticholic acid - 20 mg |
| | | Outer layer - enteric coating |
| Embodiment 24 | Coated multilayer tablet with modified release and extended release of UDCA | Core - ursodeoxycholic acid - 1000 mg |
| | | STOP-layer |
| | | Layer No. 3 - obeticholic acid - 50 mg |
| | | Outer layer - film non-enteric coating |
| Embodiment 25 | Coated multilayer tablet with modified release | Core - ursodeoxycholic acid - 100 mg |
| | | Enteric coating |
| | | Layer No. 3 - obeticholic acid - 1 mg |
| | | Outer layer - film non-enteric coating |
| Embodiment 26 | Coated multilayer tablet with modified release and extended release of UDCA | Core - ursodeoxycholic acid - 100 mg |
| | | Enteric coating |
| | | Layer No. 3 - obeticholic acid - 2.5 mg |
| | | Outer layer - film non-enteric coating |
| Embodiment 27 | Coated multilayer tablet with modified release | Core - ursodeoxycholic acid - 100 mg |
| | | Enteric coating |
| | | Layer No. 3 - obeticholic acid - 5 mg |
| | | Outer layer - film non-enteric coating |
| Embodiment 28 | Coated multilayer tablet with modified release and extended release of UDCA | Core - ursodeoxycholic acid - 100 mg |
| | | Enteric coating |
| | | Layer No. 3 - obeticholic acid - 10 mg |
| | | Outer layer - film non-enteric coating |
| Embodiment 29 | Coated multilayer tablet with modified release | Core - ursodeoxycholic acid - 100 mg |
| | | Enteric coating |
| | | Layer No. 3 - obeticholic acid - 20 mg |
| | | Outer layer - film non-enteric coating |
| Embodiment 30 | Coated multilayer tablet with modified release and extended release of UDCA | Core - ursodeoxycholic acid - 100 mg |
| | | Enteric coating |
| | | Layer No. 3 - obeticholic acid - 50 mg |
| | | Outer layer - film non-enteric coating |
| Embodiment 31 | Coated multilayer tablet with modified release | Core - ursodeoxycholic acid - 250 mg |
| | | Enteric coating |
| | | Layer No. 3 - obeticholic acid - 1 mg |
| | | Outer layer - film non-enteric coating |
| Embodiment 32 | Coated multilayer tablet with modified release and extended release of UDCA | Core - ursodeoxycholic acid - 250 mg |
| | | Enteric coating |
| | | Layer No. 3 - obeticholic acid - 2.5 mg |
| | | Outer layer - film non-enteric coating |
| Embodiment 33 | Coated multilayer tablet with modified release | Core - ursodeoxycholic acid - 250 mg |
| | | Enteric coating |
| | | Layer No. 3 - obeticholic acid - 5 mg |
| | | Outer layer - film non-enteric coating |
| Embodiment 34 | Coated multilayer tablet with modified release and extended release of UDCA | Core - ursodeoxycholic acid - 250 mg |
| | | Enteric coating |
| | | Layer No. 3 - obeticholic acid - 10 mg |
| | | Outer layer - film non-enteric coating |
| Embodiment 35 | Coated multilayer tablet with modified release | Core - ursodeoxycholic acid - 250 mg |
| | | Enteric coating |
| | | Layer No. 3 - obeticholic acid - 20 mg |
| | | Outer layer - film non-enteric coating |
| Embodiment 36 | Coated multilayer tablet with modified release and extended release of UDCA | Core - ursodeoxycholic acid - 250 mg |
| | | Enteric coating |
| | | Layer No. 3 - obeticholic acid - 50 mg |
| | | Outer layer - film non-enteric coating |
| Embodiment 37 | Coated multilayer tablet with modified release | Core - ursodeoxycholic acid - 500 mg |
| | | Enteric coating |
| | | Layer No. 3 - obeticholic acid - 1 mg |
| | | Outer layer - film non-enteric coating |
| Embodiment 38 | Coated multilayer tablet with modified release and extended release of UDCA | Core - ursodeoxycholic acid - 500 mg |
| | | Enteric coating |
| | | Layer No. 3 - obeticholic acid - 2.5 mg |
| | | Outer layer - film non-enteric coating |
| Embodiment 39 | Coated multilayer tablet with modified release | Core - ursodeoxycholic acid - 500 mg |
| | | Enteric coating |
| | | Layer No. 3 - obeticholic acid - 5 mg |
| | | Outer layer - film non-enteric coating |
| Embodiment 40 | Coated multilayer tablet with modified release and extended release of UDCA | Core - ursodeoxycholic acid - 500 mg |
| | | Enteric coating |
| | | Layer No. 3 - obeticholic acid - 10 mg |
| | | Outer layer - film non-enteric coating |
| Embodiment 41 | Coated multilayer tablet with modified release | Core - ursodeoxycholic acid - 500 mg |
| | | Enteric coating |
| | | Layer No. 3 - obeticholic acid - 20 mg |
| | | Outer layer - film non-enteric coating |
| Embodiment 42 | Coated multilayer tablet with modified release and extended release of UDCA | Core - ursodeoxycholic acid - 500 mg |
| | | Enteric coating |
| | | Layer No. 3 - obeticholic acid - 5 mg |
| | | Outer layer - film non-enteric coating |
| Embodiment 43 | Coated multilayer tablet with modified release | Core - ursodeoxycholic acid - 1000 mg |
| | | Enteric coating |
| | | Layer No. 3 - obeticholic acid - 1 mg |
| | | Outer layer - film non-enteric coating |
| Embodiment 44 | Coated multilayer tablet with modified release and extended release of UDCA | Core - ursodeoxycholic acid - 1000 mg |
| | | Enteric coating |
| | | Layer No. 3 - obeticholic acid - 2.5 mg |
| | | Outer layer - film non-enteric coating |
| Embodiment 45 | Coated multilayer tablet with modified release | Core - ursodeoxycholic acid - 1000 mg |
| | | Enteric coating |
| | | Layer No. 3 - obeticholic acid - 5 mg |
| | | Outer layer - film non-enteric coating |
| Embodiment 46 | Coated multilayer tablet with modified release and extended release of UDCA | Core - ursodeoxycholic acid - 1000 mg |
| | | Enteric coating |
| | | Layer No. 3 - obeticholic acid - 10 mg |
| | | Outer layer - film non-enteric coating |
| Embodiment 47 | Coated multilayer tablet with modified release | Core - ursodeoxycholic acid - 1002 mg |
| | | Enteric coating |
| | | Layer No. 3 - obeticholic acid - 20 mg |
| | | Outer layer - film non-enteric coating |
| Embodiment 48 | Coated multilayer tablet with modified release and extended release of UDCA | Core - ursodeoxycholic acid - 1000 mg |
| | | Enteric coating |
| | | Layer No. 3 - obeticholic acid - 50 mg |
| | | Outer layer - film non-enteric coating |

According to some embodiments of the present disclosure, the claimed solid dosage form may have the following composition:

### Example 50

| Unit Composition UDCA+OCA combination | |
|---|---|
| Strength | 500+25 |

| Ingredient | Quantity/Tablet (in mg) |
|---|---|
| UDCA Core | |
| Ursodeoxycholic acid | 500 |
| Disodium Phosphate | 50 |
| Polyvinylpyrrolidone | 24 |
| Microcrystalline Cellulose | 150 |
| Magnesium Stearate | 10 |
| Talc | 6 |
| Hydroxypropylmethylcellulose | 14 |
| PEG 6000 | 10 |
| Titanium Dioxide | 2 |
| Cellulose Acetate Phthalate | 24 |
| Acetylated Monoglycerides | 4 |
| STOP Layer | |
| Microcrystalline Cellulose | 12 |
| Starch maize | 6 |
| Crospovidone | 4 |
| Polyvinylpyrrolidone | 4 |
| OCA Layer | |
| Obeticholic acid | 25 |
| Microcrystalline Cellulose | 29 |
| Sodium Starch Glycollate | 8 |
| Colloidal Silicon Dioxide | 2 |
| Magnesium Stearate | 4 |
| Gastric acid soluble coating | |
| Opadry (Ready Film coating material) | 12 |

### Example 51

| Unit Composition UDCA+OCA combination | |
|---|---|
| Strength | 100+5 |

| Ingredient | Quantity/Tablet (in mg) |
|---|---|
| UDCA Core | |
| Ursodeoxycholic acid | 100 |
| L Arginine | 10 |
| Polyvinylpyrrolidone | 10 |
| Microcrystalline Cellulose | 40 |
| Magnesium Stearate | 2 |
| Talc | 2 |
| Hydroxypropylmethylcellulose | 6 |
| PEG 6000 | 2 |
| Titanium Dioxide | 1 |
| Eudragit L100 | 6 |
| Acetylated Monoglycerides | 1 |
| STOP Layer | |
| Microcrystalline Cellulose | 17 |
| Starch maize | 6 |
| Crospovidone | 2 |
| Polyvinylpyrrolidone | 3 |
| OCA Layer | |
| Obeticholic acid | 5 |
| Microcrystalline Cellulose | 20 |
| Sodium Starch Glycollate | 2 |
| Colloidal Silicon Dioxide | 1 |
| Magnesium Stearate | 2 |
| Gastric acid soluble coating | |
| Opadry (Ready Film coating material) | 12 |

### Example 52

| Unit Composition UDCA+OCA combination | |
|---|---|
| Strength | 200+10 |

| Ingredient | Quantity/Tablet (in mg) |
|---|---|
| UDCA Core | |
| Ursodeoxycholic acid | 200 |
| Sodium Maleate | 20 |
| Polyvinylpyrrolidone | 20 |
| Microcrystalline Cellulose | 80 |
| Magnesium Stearate | 4 |
| Talc | 4 |
| Ethyl Cellulose | 12 |
| PEG 6000 | 4 |
| Titanium Dioxide | 2 |
| Eudragit L100 | 12 |
| Acetylated Monoglycerides | 2 |
| STOP Layer | |
| Microcrystalline Cellulose | 30 |
| Starch maize | 10 |
| Crospovidone | 4 |
| Polyvinylpyrrolidone | 6 |
| OCA Layer | |
| Obeticholic acid | 20 |
| Microcrystalline Cellulose | 36 |
| Sodium Starch Glycollate | 4 |
| Colloidal Silicon Dioxide | 2 |
| Magnesium Stearate | 4 |
| Gastric acid soluble coating | |
| Opadry (Ready Film coating material) | 24 |

### Example 53

| | |
|---|---|
| Unit Composition UDCA+OCA combination | |
| Strength | 100+5 |

| Ingredient | Quantity/Tablet (in mg) |
|---|---|
| UDCA Core | |
| Ursodeoxycholic acid | 100 |
| Disodium Phosphate | 10 |
| Polyvinylpyrrolidone | 10 |
| Microcrystalline Cellulose | 40 |
| Magnesium Stearate | 2 |
| Talc | 2 |
| Hydroxy Ethyl Cellulose | 6 |
| PEG 6000 | 2 |
| Titanium Dioxide | 1 |
| Eudragit L55D (EQ to dried solid content) | 6 |
| Acetylated Monoglycerides | 1 |
| STOP Layer | |
| Microcrystalline Cellulose | 17 |
| Starch maize | 6 |
| Crospovidone | 2 |
| Polyvinylpyrrolidone | 3 |
| OCA Layer | |
| Obeticholic acid | 5 |
| Microcrystalline Cellulose | 20 |
| Sodium Starch Glycollate | 2 |
| Colloidal Silicon Dioxide | 1 |
| Magnesium Stearate | 2 |
| Gastric acid soluble coating | |
| Opadry (Ready Film coating material) | 12 |

### Example 54

| Unit Composition UDCA+OCA combination | |
|---|---|
| Strength | 250+12.5 |

| Ingredient | Quantity/Tablet (in mg) |
|---|---|
| UDCA Core | |
| Ursodeoxycholic acid | 250 |
| Sodium Bicarbobate | 25 |
| Polyvinylpyrrolidone | 12 |
| Microcrystalline Cellulose | 75 |
| Magnesium Stearate | 5 |
| Talc | 3 |
| Hydroxypropylmethylcellulose | 7 |
| PEG 6000 | 5 |
| Titanium Dioxide | 1 |
| Hydroxypropylmethylcellulose Acetyl Succinate | 12 |
| Acetylated Monoglycerides | 2 |
| STOP Layer | |
| Microcrystalline Cellulose | 3 |
| Starch maize | 1 |
| Crospovidone | 2 |
| Polyvinylpyrrolidone | 2 |
| OCA Layer | |
| Obeticholic acid | 12.5 |
| Microcrystalline Cellulose | 20 |
| Sodium Starch Glycollate | 4 |
| Colloidal Silicon Dioxide | 0.5 |
| Magnesium Stearate | 2 |
| Gastric acid soluble coating | |
| Opadry (Ready Film coating material) | 6 |

### Example 55

| Unit Composition UDCA+OCA combination | |
|---|---|
| Strength | 1000+50 |

| Ingredient | Quantity/Tablet (in mg) |
|---|---|
| UDCA Core | |
| Ursodeoxycholic acid | 1000 |
| Sodium Bicarbobate | 100 |
| Polyvinylpyrrolidone | 30 |
| Microcrystalline Cellulose | 300 |
| Magnesium Stearate | 20 |
| Talc | 13 |
| Hydroxypropylmethylcellulose | 28 |
| PEG 6000 | 20 |
| Titanium Dioxide | 5 |
| Hydroxypropylmethylcellulose Phthalate | 50 |
| Acetylated Monoglycerides | 6 |
| STOP Layer | |
| Microcrystalline Cellulose | 30 |
| Starch maize | 15 |
| Crospovidone | 8 |
| Polyvinylpyrrolidone | 8 |
| OCA Layer | |
| Obeticholic acid | 50 |
| Microcrystalline Cellulose | 80 |
| Sodium Starch Glycollate | 8 |
| Colloidal Silicon Dioxide | 3 |
| Magnesium Stearate | 2 |
| Gastric acid soluble coating | |
| Opadry (Ready Film coating material) | 24 |

Examples of the production of multilayer tablets according to the invention are shown below in the Examples. The number of the components in the multilayer tablets is shown in the Tables provided in each example.

### Example 56

The amount of each component in one tablet is shown in the Table below. For the purpose of manufacturing one hundred tablets, the mass of each component was 100-fold the mass specified in the Table.

Ursodeoxycholic acid, L-arginine, 50% microcrystalline cellulose, 50% hydroxypropylmethylcellulose and holyvinylpyrrolidone are dry mixed and wet granulated in an appropriate granulator with sufficient purified water.

The wet granules are dried, milled, and blended with the remaining 50% microcrystalline cellulose, remaining 50% hydroxypropylmethylcellulose, talc, magnesium stearate, PEG 6000, titanium dioxide, Eudragit L100 and acetylated monoglycerides.

The final granule blend is compressed into tablet cores.

Microcrystalline cellulose, starch maize, crospovidone, and polyvinylpyrrolidone are added slowly to purified water and mixing is continued until the components are fully dispersed.

The dispersion is sprayed onto the tablet cores in a conventional coating pan until proper amount of STOP layer is deposited on the tablet cores.

Obeticholic acid is dissolved in purified water. After thorough mixing, microcrystalline cellulose, sodium starch glycollate, colloidal silicon dioxide, and magnesium stearate are added slowly, and mixing is continued until the components are fully dispersed. The suspension is sprayed onto the tablet cores covered with STOP layer in a conventional coating pan until the proper amount of OCA layer is deposited.

Opadry is added slowly to purified water and mixing is continued until Opadry is fully dispersed. The solution is sprayed onto the tablet cores covered with STOP layer and OCA layer in a conventional coating pan until proper amount of Opadry is deposited on the tablets. A sample of the tablets is tested for gastric resistance and the coating stopped if the tablets pass the test.

| Single tablet composition | |
|---|---|
| Ingredients UDCA+OCA, mg | 100+1 |

| Ingredient | Qty/Tablet (in mg) |
|---|---|
| UDCA Core | |
| Ursodeoxycholic acid | 100 |
| L Arginine | 10 |
| Polyvinylpyrrolidone | 10 |
| Microcrystalline Cellulose | 40 |
| Magnesium Stearate | 2 |
| Talc | 2 |
| Hydroxypropylmethylcellulose | 6 |
| PEG 6000 | 2 |
| Titanium Dioxide | 1 |
| Eudragit L100 | 6 |
| Acetylated Monoglycerides | 1 |
| STOP Layer | |
| Microcrystalline Cellulose | 17 |
| Starch maize | 6 |
| Crospovidone | 2 |
| Polyvinylpyrrolidone | 3 |
| OCA Layer | |
| Obeticholic acid | 1 |
| Microcrystalline Cellulose | 20 |
| Sodium Starch Glycollate | 2 |
| Colloidal Silicon Dioxide | 1 |
| Magnesium Stearate | 2 |
| Gastric acid soluble layer | |
| Opadry (Ready Film coating material) | 12 |

### Example 57

The amount of each component in one tablet is shown in the Table below. For the purpose of manufacturing one hundred tablets, the mass of each component was 100-fold the mass specified in the Table.

Ursodeoxycholic acid, L-arginine, 50% microcrystalline cellulose, 50% hydroxypropylmethylcellulose and holyvinylpyrrolidone are dry mixed and wet granulated in an appropriate granulator with sufficient purified water.

The wet granules are dried, milled, and blended with the remaining 50% microcrystalline cellulose, remaining 50% hydroxypropylmethylcellulose, talc, magnesium stearate, PEG 6000, titanium dioxide, Eudragit L100 and acetylated monoglycerides.

The final granule blend is compressed into tablet cores.

Microcrystalline cellulose, starch maize, crospovidone, and polyvinylpyrrolidone are added slowly to purified water and mixing is continued until the components are fully dispersed.

The dispersion is sprayed onto the tablet cores in a conventional coating pan until proper amount of STOP layer is deposited on the tablet cores.

Obeticholic acid is dissolved in purified water. After thorough mixing, microcrystalline cellulose, sodium starch glycollate, colloidal silicon dioxide, and magnesium stearate are added slowly, and mixing is continued until the components are fully dispersed. The suspension is sprayed onto the tablet cores covered with STOP layer in a conventional coating pan until the proper amount of OCA layer is deposited.

Opadry is added slowly to purified water and mixing is continued until Opadry is fully dispersed. The solution is sprayed onto the tablet cores covered with STOP layer and OCA layer in a conventional coating pan until proper amount of Opadry is deposited on the tablets. A sample of the tablets is tested for gastric resistance and the coating stopped if the tablets pass the test.

| Single tablet composition | |
|---|---|
| Ingredients UDCA+OCA, mg | 150+1,5 |

| Ingredient | Qty/Tablet (in mg) |
|---|---|
| UDCA Core | |
| Ursodeoxycholic acid | 150 |
| L Arginine | 20 |
| Polyvinylpyrrolidone | 20 |
| Microcrystalline Cellulose | 80 |
| Magnesium Stearate | 4 |
| Talc | 4 |
| Hydroxypropylmethylcellulose | 12 |
| PEG 6000 | 4 |
| Titanium Dioxide | 2 |
| Eudragit L100 | 12 |
| Acetylated Monoglycerides | 2 |
| STOP Layer | |
| Microcrystalline Cellulose | 30 |
| Starch maize | 10 |
| Crospovidone | 4 |
| Polyvinylpyrrolidone | 6 |
| OCA Layer | |
| Obeticholic acid | 1,5 |
| Microcrystalline Cellulose | 25 |
| Sodium Starch Glycollate | 4 |
| Colloidal Silicon Dioxide | 2 |
| Magnesium Stearate | 4 |
| Gastric acid soluble layer | |
| Opadry (Ready Film coating material) | 28 |

### Example 58

The amount of each component in one tablet is shown in the Table below. For the purpose of manufacturing one hundred tablets, the mass of each component was 100-fold the mass specified in the Table.

Ursodeoxycholic acid, L-arginine, 50% microcrystalline cellulose, 50% hydroxypropylmethylcellulose and holyvinylpyrrolidone are dry mixed and wet granulated in an appropriate granulator with sufficient purified water.

The wet granules are dried, milled, and blended with the remaining 50% microcrystalline cellulose, remaining 50% hydroxypropylmethylcellulose, talc, magnesium stearate, PEG 6000, titanium dioxide, Eudragit L100 and acetylated monoglycerides.

The final granule blend is compressed into tablet cores.

Microcrystalline cellulose, starch maize, crospovidone, and polyvinylpyrrolidone are added slowly to purified water and mixing is continued until the components are fully dispersed.

The dispersion is sprayed onto the tablet cores in a conventional coating pan until proper amount of STOP layer is deposited on the tablet cores.

Obeticholic acid is dissolved in purified water. After thorough mixing, microcrystalline cellulose, sodium starch glycollate, colloidal silicon dioxide, and magnesium stearate are added slowly, and mixing is continued until the components are fully dispersed. The suspension is sprayed onto the tablet cores covered with STOP layer in a conventional coating pan until the proper amount of OCA layer is deposited.

Opadry is added slowly to purified water and mixing is continued until Opadry is fully dispersed. The solution is sprayed onto the tablet cores covered with STOP layer and OCA layer in a conventional coating pan until proper amount of Opadry is deposited on the tablets. A sample of the tablets is tested for gastric resistance and the coating stopped if the tablets pass the test.

| Single tablet composition | |
|---|---|
| Ingredients UDCA+OCA, mg | 200+2,5 |

| Ingredient | Qty/Tablet (in mg) |
|---|---|
| UDCA Core | |
| Ursodeoxycholic acid | 200 |
| L Arginine | 30 |
| Polyvinylpyrrolidone | 25 |
| Microcrystalline Cellulose | 150 |
| Magnesium Stearate | 10 |
| Talc | 6 |
| Hydroxypropylmethylcellulose | 15 |
| PEG 6000 | 10 |
| Titanium Dioxide | 2 |
| Eudragit L100 | 24 |
| Acetylated Monoglycerides | 4 |
| STOP Layer | |
| Microcrystalline Cellulose | 36 |
| Starch maize | 14 |
| Crospovidone | 6 |
| Polyvinylpyrrolidone | 6 |
| OCA Layer | |
| Obeticholic acid | 2,5 |
| Microcrystalline Cellulose | 30 |
| Sodium Starch Glycollate | 6 |
| Colloidal Silicon Dioxide | 4 |
| Magnesium Stearate | 6 |
| Gastric acid soluble layer | |
| Opadry (Ready Film coating material) | 36 |

### Example 59

The amount of each component in one tablet is shown in the Table below. For the purpose of manufacturing one hundred tablets, the mass of each component was 100-fold the mass specified in the Table.

Ursodeoxycholic acid, L-arginine, 50% microcrystalline cellulose, 50% hydroxypropylmethylcellulose and holyvinylpyrrolidone are dry mixed and wet granulated in an appropriate granulator with sufficient purified water.

The wet granules are dried, milled, and blended with the remaining 50% microcrystalline cellulose, remaining 50% hydroxypropylmethylcellulose, talc, magnesium stearate, PEG 6000, titanium dioxide, Eudragit L100 and acetylated monoglycerides.

The final granule blend is compressed into tablet cores.

Microcrystalline cellulose, starch maize, crospovidone, and polyvinylpyrrolidone are added slowly to purified water and mixing is continued until the components are fully dispersed.

The dispersion is sprayed on to the tablet cores in a conventional coating pan until proper amount of STOP layer is deposited on the tablet cores.

Obeticholic acid is dissolved in purified water. After thorough mixing, microcrystalline cellulose, sodium starch glycollate, colloidal silicon dioxide, and magnesium stearate are added slowly, and mixing is continued until the components are fully dispersed. The suspension is sprayed onto the tablet cores covered with STOP layer in a conventional coating pan until the proper amount of OCA layer is deposited.

Opadry is added slowly to purified water and mixing is continued until Opadry is fully dispersed. The solution is sprayed onto the tablet cores covered with STOP layer and OCA layer in a conventional coating pan until proper amount of Opadry is deposited on the tablets. A sample of the tablets is tested for gastric resistance and the coating stopped if the tablets pass the test.

| Single tablet composition | |
|---|---|
| Ingredients UDCA+OCA, mg | 250+5 |

| Ingredient | Qty/Tablet (in mg) |
|---|---|
| UDCA Core | |
| Ursodeoxycholic acid | 250 |
| L Arginine | 40 |
| Polyvinylpyrrolidone | 30 |
| Microcrystalline Cellulose | 300 |
| Magnesium Stearate | 20 |
| Talc | 12 |
| Hydroxypropylmethylcellulose | 28 |
| PEG 6000 | 20 |
| Titanium Dioxide | 5 |
| Eudragit L100 | 44 |
| Acetylated Monoglycerides | 6 |
| STOP Layer | |
| Microcrystalline Cellulose | 40 |
| Starch maize | 20 |
| Crospovidone | 8 |
| Polyvinylpyrrolidone | 8 |
| OCA Layer | |
| Obeticholic acid | 5 |
| Microcrystalline Cellulose | 40 |
| Sodium Starch Glycollate | 8 |
| Colloidal Silicon Dioxide | 4 |
| Magnesium Stearate | 8 |
| Gastric acid soluble layer | |
| Opadry (Ready Film coating material) | 44 |

### Example 60

The amount of each component in one tablet is shown in the Table below. For the purpose of manufacturing one hundred tablets, the mass of each component was 100-fold the mass specified in the Table.

Ursodeoxycholic acid, L-arginine, 50% microcrystalline cellulose, 50% hydroxypropylmethylcellulose and holyvinylpyrrolidone are dry mixed and wet granulated in an appropriate granulator with sufficient purified water.

The wet granules are dried, milled, and blended with the remaining 50% microcrystalline cellulose, remaining 50% hydroxypropylmethylcellulose, talc, magnesium stearate, PEG 6000, titanium dioxide, Eudragit L100 and acetylated monoglycerides.

The final granule blend is compressed into tablet cores.

Microcrystalline cellulose, starch maize, crospovidone, and polyvinylpyrrolidone are added slowly to purified water and mixing is continued until the components are fully dispersed.

The dispersion is sprayed onto the tablet cores in a conventional coating pan until proper amount of STOP layer is deposited on the tablet cores.

Obeticholic acid is dissolved in purified water. After thorough mixing, microcrystalline cellulose, sodium starch glycollate, colloidal silicon dioxide, and magnesium stearate are added slowly, and mixing is continued until the components are fully dispersed. The suspension is sprayed onto the tablet cores covered with STOP layer in a conventional coating pan until the proper amount of OCA layer is deposited.

Opadry is added slowly to purified water and mixing is continued until Opadry is fully dispersed. The solution is sprayed onto the tablet cores covered with STOP layer and OCA layer in a conventional coating pan until proper amount of Opadry is deposited on the tablets. A sample of the tablets is tested for gastric resistance and the coating stopped if the tablets pass the test.

| Single tablet composition | |
|---|---|
| Ingredients UDCA+OCA, mg | 500+5 |

| Ingredient | Qty/Tablet (in mg) |
|---|---|
| UDCA Core | |
| Ursodeoxycholic acid | 500 |
| L Arginine | 45 |
| Polyvinylpyrrolidone | 34 |
| Microcrystalline Cellulose | 350 |
| Magnesium Stearate | 20 |
| Talc | 12 |
| Hydroxypropylmethylcellulose | 28 |
| PEG 6000 | 20 |
| Titanium Dioxide | 5 |
| Eudragit L100 | 44 |
| Acetylated Monoglycerides | 6 |
| STOP Layer | |
| Microcrystalline Cellulose | 45 |
| Starch maize | 25 |
| Crospovidone | 8 |
| Polyvinylpyrrolidone | 8 |
| OCA Layer | |
| Obeticholic acid | 5 |
| Microcrystalline Cellulose | 60 |
| Sodium Starch Glycollate | 8 |
| Colloidal Silicon Dioxide | 8 |
| Magnesium Stearate | 8 |
| Gastric acid soluble layer | |
| Opadry (Ready Film coating material) | 48 |

### Example 61

The amount of each component in one tablet is shown in the Table below. For the purpose of manufacturing one hundred tablets, the mass of each component was 100-fold the mass specified in the Table.

Ursodeoxycholic acid, L-arginine, 50% microcrystalline cellulose, 50% hydroxypropylmethylcellulose and holyvinylpyrrolidone are dry mixed and wet granulated in an appropriate granulator with sufficient purified water.

The wet granules are dried, milled, and blended with the remaining 50% microcrystalline cellulose, remaining 50% hydroxypropylmethylcellulose, talc, magnesium stearate, PEG 6000, titanium dioxide, Eudragit L100 and acetylated monoglycerides.

The final granule blend is compressed into tablet cores.

Microcrystalline cellulose, starch maize, crospovidone, and polyvinylpyrrolidone are added slowly to purified water and mixing is continued until the components are fully dispersed.

The dispersion is sprayed on to the tablet cores in a conventional coating pan until proper amount of STOP layer is deposited onthe tablet cores.

Obeticholic acid is dissolved in purified water. After thorough mixing, microcrystalline cellulose, sodium starch glycollate, colloidal silicon dioxide, and magnesium stearate are added slowly, and mixing is continued until the components are fully dispersed. The suspension is sprayed onto the tablet cores covered with STOP layer in a conventional coating pan until the proper amount of OCA layer is deposited.

Opadry is added slowly to purified water and mixing is continued until Opadry is fully dispersed. The solution is sprayed onto the tablet cores covered with STOP layer and OCA layer in a conventional coating pan until proper amount of Opadry is deposited on the tablets. A sample of the tablets is tested for gastric resistance and the coating stopped if the tablets pass the test.

| Single tablet composition | |
|---|---|
| Ingredients UDCA+OCA, mg | 750+10 |
| Ingredient | Qty/Tablet (in mg) |
| UDCA Core | |
| Ursodeoxycholic acid | 750 |
| L Arginine | 50 |
| Polyvinylpyrrolidone | 40 |
| Microcrystalline Cellulose | 400 |
| Magnesium Stearate | 25 |
| Talc | 17 |
| Hydroxypropylmethylcellulose | 35 |
| PEG 6000 | 26 |
| Titanium Dioxide | 7 |
| Eudragit L100 | 50 |
| Acetylated Monoglycerides | 10 |
| STOP Layer | |
| Microcrystalline Cellulose | 55 |
| Starch maize | 30 |
| Crospovidone | 10 |
| Polyvinylpyrrolidone | 10 |
| OCA Layer | |
| Obeticholic acid | 10 |
| Microcrystalline Cellulose | 80 |
| Sodium Starch Glycollate | 10 |
| Colloidal Silicon Dioxide | 10 |
| Magnesium Stearate | 10 |
| Gastric acid soluble layer | |
| Opadry (Ready Film coating material) | 60 |

### Example 62

The amount of each component in one tablet is shown in the Table below. For the purpose of manufacturing one hundred tablets, the mass of each component was 100-fold the mass specified in the Table.

Ursodeoxycholic acid, L-arginine, 50% microcrystalline cellulose, 50% hydroxypropylmethylcellulose and holyvinylpyrrolidone are dry mixed and wet granulated in an appropriate granulator with sufficient purified water.

The wet granules are dried, milled, and blended with the remaining 50% microcrystalline cellulose, remaining 50% hydroxypropylmethylcellulose, talc, magnesium stearate, PEG 6000, titanium dioxide, Eudragit L100 and acetylated monoglycerides.

The final granule blend is compressed into tablet cores.

Microcrystalline cellulose, starch maize, crospovidone, and polyvinylpyrrolidone are added slowly to purified water and mixing is continued until the components are fully dispersed.

The dispersion is sprayed onto the tablet cores in a conventional coating pan until proper amount of STOP layer is deposited on the tablet cores.

Obeticholic acid is dissolved in purified water. After thorough mixing, microcrystalline cellulose, sodium starch glycollate, colloidal silicon dioxide, and magnesium stearate are added slowly, and mixing is continued until the components are fully dispersed. The suspension is sprayed onto the tablet cores covered with STOP layer in a conventional coating pan until the proper amount of OCA layer is deposited.

Opadry is added slowly to purified water and mixing is continued until Opadry is fully dispersed. The solution is sprayed onto the tablet cores covered with STOP layer and OCA layer in a conventional coating pan until proper amount of Opadry is deposited on the tablets. A sample of the tablets is tested for gastric resistance and the coating stopped if the tablets pass the test.

| Single tablet composition | |
|---|---|
| Ingredients UDCA+OCA, mg | 1000+10 |
| Ingredient | Qty/Tablet (in mg) |
| UDCA Core | |
| Ursodeoxycholic acid | 1000 |
| L Arginine | 70 |
| Polyvinylpyrrolidone | 50 |
| Microcrystalline Cellulose | 500 |
| Magnesium Stearate | 35 |
| Talc | 24 |
| Hydroxypropylmethylcellulose | 45 |
| PEG 6000 | 40 |
| Titanium Dioxide | 10 |
| Eudragit L100 | 70 |
| Acetylated Monoglycerides | 15 |
| STOP Layer | |
| Microcrystalline Cellulose | 70 |
| Starch maize | 40 |
| Crospovidone | 12 |
| Polyvinylpyrrolidone | 12 |
| OCA Layer | |
| Obeticholic acid | 10 |
| Microcrystalline Cellulose | 100 |
| Sodium Starch Glycollate | 20 |
| Colloidal Silicon Dioxide | 15 |
| Magnesium Stearate | 15 |
| Gastric acid soluble layer | |
| Opadry (Ready Film coating material) | 80 |

In order for the patient to receive his daily dose of UDCA and OCA, he can take, for example, several tablets per day, specified in Examples 56-60 (for example, three or four tablets from Example 2), or one tablet specified in Examples 61-62.

The dissolution data for one of the representatives of disclosed solid dosage forms is mentioned below (Table 17) for both OCA and UDCA actives in the specific media:

**Table 17**

| Values of the release rates of OCA and UDCA upon dissolution of the claimed solid dosage form, in % | | | | | | | |
|---|---|---|---|---|---|---|---|
| Percent Drug released in minutes | | | | | | | |
| Time (in mins.) | 15 | 30 | 45 | 60 | 120 | 240 | 300 |
| OCA | 52 | 65 | 75 | 99 | 100 | 100 | 100 |
| UDCA | 0 | 0 | 0 | 0 | 23 | 48 | 99 |

A double-blind clinical study on the effectiveness of the combined pharmaceutical composition comprising UDCA and OCA compared to UDCA was conducted.

To study the effectiveness, the tablet embodiments No. 15 and No. 16 were used.

| | | |
|---|---|---|
| Embodiment 15 | Coated multilayer tablet with modified release | Core - ursodeoxycholic acid - 500 mg |
| | | STOP-layer |
| | | Layer No. 3 - obeticholic acid - 5 mg |
| | | Outer layer - enteric coating |
| Embodiment 16 | Coated multilayer tablet with modified release and extended release of UDCA | Core - ursodeoxycholic acid - 500 mg |
| | | STOP-layer |
| | | Layer No. 3 - obeticholic acid - 10 mg |
| | | Outer layer - film non-enteric coating |

A study on the effectiveness of the various treatment regimens for the patients with PBC was conducted. A total of three treatment regimens were used in the study:
1) UDCA monocomposition at a dose of 15 mg/kg/day.
2) Combined pharmaceutical composition in the pharmaceutical form with the modified release and at a dose of UDCA 15 mg/kg + OCA 5 mg per day.
3) Combined pharmaceutical composition in the pharmaceutical form with the modified release and at a dose of UDCA 15 mg/kg + OCA 10 mg per day.

The studies were carried out for 12 months, during which the quality of the treatment was assessed by measuring biochemical and molecular biological markers (see above).

The evaluation of the effectiveness of the treatment was carried out on the basis of the Paris2 system, according to which the criteria for a satisfactory treatment are:
• ALT level ≤ 3-fold upper limit of normal (ULN);
• AST level ≤ 2-fold ULN;
• normal bilirubin level.

**Table 18**

| Effectiveness of the PBC treatment using various regimens | | | |
|---|---|---|---|
| | Total number of patients | Number of patients with positive response to treatment (number) | Number of patients with positive response to treatment (%) |
| UDCA 15 mg/kg | 68 | 40 | 58.82 |
| UDCA 15 mg/kg + OCA 5 mg | 65 | 56 | 86.15 |
| UDCA 15 mg/kg + OCA 10 mg | 63 | 58 | 92.06 |

According to the data above, the treatment regimen using a combined pharmaceutical composition in the pharmaceutical form with the modified release and at the dose of UDCA 15 mg/kg + OCA 10 mg per day, was found to be the most effective (92.06% of the patients had a positive result of the treatment). In second place, the treatment regimen using the combined pharmaceutical composition in the pharmaceutical form with the modified release and at the dose of UDCA 15 mg/kg + OCA 5 mg per day (86.15% of the patients had a positive result of the treatment). The regimen with the use of UDCA monocomposition had the lowest effectiveness - 58.82%.

A multi-center, randomized, double-blind, placebo-controlled, multi-dose, parallel group trial was conducted to determine efficacy of the treatment of primary biliary cholangitis (PBC) with multilayer tablets comprising obeticholic acid (OCA) and ursodeoxycholic acid (UDCA).

165 male and female aged 18-75 years with diagnosed PBC on a stable dose of UDCA for at least 6 months prior to screening were enrolled in the trial. Weight of the patients was in a range of 70 to 90 kg. The diagnosis of PBC was based on the presence of at least 2 of 3 key criteria: (1) an AMA titer higher than 1:40, (2) abnormal alkaline phosphatase (Alk-p) levels [≥1.5 X upper limit of normal (ULN)], and (3) liver histology compatible with PBC.

The patients were randomized into placebo group, 1000 mg UDCA + 10 mg OCA (2 multi-layered tablets 500 mg UDCA + 5 mg OCA per day) and 1000 mg UDCA + 20 mg (2 multi-layered tablets 500 mg UDCA + 10 mg OCA per day) for 12 weeks. A multilayer tablet 500 mg UDCA + 5 mg OCA comprised core containing 500 mg of UDCA, STOP-layer, layer No. 3 containing 5 mg of OCA and outer layer being film non-enteric coating. Similarly, multilayer tablet 500 mg UDCA + 10 mg OCA comprised core containing 500 mg of UDCA, STOP-layer, layer No. 3 containing 10 mg of OCA and outer layer being film non-enteric coating.

The patients were examined for the change in serum alkaline phosphatase (ALP) from Baseline to Day 85/ET.

**Table 19**

| ALP (U/L) at Baseline and Day 85 | | | |
|---|---|---|---|
| | Placebo | UDCA + 10 mq OCA | UDCA + 20 mg OCA |
| Baseline | | | |
| Mean (SD) | 275,2 (102,7) | 294,4 (149,4) | 290,0 (123,6) |
| Median | 249,5 | 234,8 | 255,8 |

| Day 85 | | | |
|---|---|---|---|
| Mean (SD) | 270,7 (118,7) | 219,0 (113,5) | 225,0 (169,1) |
| Median | 234,5 | 177,5 | 187,5 |
| Mean (SD) percent change | -2,6 (12,4) | -23,3 (17,1) | -24,0 (18,8) |
| Median percent change | -3,2 | -21,0 | -27,8 |
| P-value | -- | <0,0001 | <0,0001 |

Table shows that compared with the placebo group, the groups taking UDCA + 10 mg OCA and UDCA + 20 mg OCA had a greater mean percent decrease in their ALP (-23.7%±17.8%, - 24.7%±17.9%, respectively, vs 2.6%±12.5%, P<0.0001). Hence, the trial conducted confirms positive impact of UDCA + OCA combination on the PBS course in terms of changes in serum ALP.

A randomized clinical trial was conducted to determine the clinical outcomes of the combination therapy using multilayer tablets comprising ursodeoxycholic acid and obeticholic acid compared with ursodeoxycholic acid monotherapy in patients with primary biliary cholangitis (PBC).

170 male and female aged 18-75 years with diagnosed PBC were enrolled in the trial. Average weight of the patients was 78.9 kg. The diagnosis of PBC was based on the presence of at least 2 of 3 key criteria: (1) an AMA titer higher than 1:40, (2) abnormal alkaline phosphatase (Alk-p) levels [≥1.5 X upper limit of normal (ULN)], and (3) liver histology compatible with PBC.

The patients were randomized into placebo group, 1000 mg UDCA + 10 mg OCA (2 multilayer tablets 500 mg UDCA + 5 mg OCA per day) and 1000 mg UDCA + 20 mg (2 multilayer tablets 500 mg UDCA + 10 mg OCA per day) for 12 weeks. A multilayer tablet 500 mg UDCA + 5 mg OCA comprised core containing 500 mg of UDCA, enteric coating, layer No. 3 containing 5 mg of OCA and outer layer being film non-enteric coating. Similarly, a multilayer tablet 500 mg UDCA + 10 mg OCA comprised core containing 500 mg of UDCA, enteric coating, layer No. 3 containing 10 mg of OCA and outer layer being film non-enteric coating.

The primary endpoints were 1) less than 1.67 times the upper limit of normal (ULN) of serum alkaline phosphatase with 15% reduction from baseline and 2) serum total bilirubin within normal limits at the completion of the trials (85 days and 12 months). Secondary outcomes included liver biochemistry parameters including serum alanine aminotransferase (ALT), aspartate aminotransferase (AST), and gamma-glutamyl transpeptidase (GGT), and conjugated bilirubin.

52% of patients in the UDCA + OCA therapy groups and 22% in the monotherapy groups met both of the endpoints. The results indicate that UDCA + OCA therapy was significantly superior to monotherapy in reducing serum ALT (MD -15.63 IU/L; 95% CI, -21.59 to -9.68), AST (MD -6.63 IU/L; 95% CI, -11.03 to -2.24), and GGT (MD - 131.30 IU/L; 95% CI, -177.52 to -85.08). However, there was no significant difference between UDCA + OCA therapy groups and monotherapy groups for reducing conjugated bilirubin (MD-0.06mg/dL; 95% CI, -0.28 to 0.15; p=0.56). Overall, the results indicated that UDCA + OCA therapy did not differ significantly from monotherapy in reducing bilirubin but was statistically significantly superior to monotherapy in reducing liver biochemical parameters.

A randomized, double-blind, placebo-controlled trial was conducted to determine efficacy of the treatment of PBC with multilayer tablets comprising obeticholic acid (OCA) and ursodeoxycholic acid (UDCA) in patients with primary biliary cirrhosis.

77 male and female aged 18-75 years with diagnosed PBC were enrolled in the trial. Average weight of the patients was 80.1 kg. The diagnosis of PBC was based on the presence of at least 2 of 3 key criteria: (1) an AMA titer higher than 1:40, (2) abnormal alkaline phosphatase (Alk-p) levels [≥2 X upper limit of normal (ULN)], and (3) liver histology compatible with PBC. In addition, total bilirubin was <2.5x ULN.

The patients were randomized into placebo group, 500 mg UDCA + 2.5 mg OCA (Group 1, 1 oral multilayer tablet per day) and 750 mg UDCA + 5 mg OCA (Group 2, 1 oral multilayer tablet per day) for the first 12 weeks. After 12 weeks, providing there were no limiting safety or tolerability concerns, the dose was up titrated to 1000 mg UDCA + 5 mg OCA (Group 1, 2 oral multilayer tablets 500 mg UDCA + 2.5 mg OCA per day) and 1500 mg UDCA + 10 mg OCA (Group 2, 2 oral multilayer tablets 750 mg UDCA + 5 mg OCA per day), and the placebo group remained on placebo, for the additional 12 weeks.

A multilayer tablet 500 mg UDCA + 2.5 mg OCA comprised core containing 500 mg of UDCA, STOP-layer, layer No. 3 containing 2/5 mg of OCA and outer layer being film non-enteric coating. Similarly, a multilayer tablet 750 mg UDCA 5 mg OCA comprised core containing 500 mg of UDCA, STOP-layer, layer No. 3 containing 10 mg of OCA and outer layer being film non-enteric coating.

The primary efficacy endpoint was change from baseline in serum ALP at week 24. Secondary endpoints included: alanine aminotransferase (ALT), aspartate aminotransferase (AST), gamma-glutamyl transferase (GGT), direct bilirubin and total bilirubin.

The 500 mg UDCA + 2.5 mg OCA dose administered in the first 12 weeks of the trial showed no meaningful biochemical response. However, while 750 mg UDCA + 5 mg OCA dose appeared to show additional improvement over 500 mg UDCA + 2.5 mg OCA, this was not statistically different from placebo. At week 12, the decrease in ALP in Group 1 was not significantly different from placebo. As well as at week 24, the decrease in ALP in Group 2 was not significantly different from placebo (LS mean [SE] difference = -78.29 [41.81] U/L, 95% CI -162.08 to 5.50; p = 0.067).

This trial demonstrated that Group 2 treatment regimen led to significant reductions in ALP, a marker of cholestasis, in patients with PSC after 24 weeks. Improvements in ALP were apparent as early as 2 weeks after the treatment initiation and were sustained throughout the treatment. At week 12, the decrease in ALP in Group 2 was significantly different from placebo (LS mean [SE] difference of -82.35 [33.39] U/L) (95% CI -149.11 to -15.59; p = 0.017). At week 24, the decrease in ALP in Group 2 was also significantly different from placebo (LS mean (SE) difference of -83.42 (40.34) U/L (95% CI -164.28 to -2.57; p = 0.043). Titration in Group 2 from 750 mg UDCA + 5 mg OCA to 1500 mg UDCA + 10 mg OCA did not lead to further reductions in ALP.

Median values of total and direct bilirubin were within the normal reference range in all treatment groups during the first 12 weeks and generally fluctuated around the respective baseline values throughout the whole treatment. Median AST and ALT values generally decreased during the first 12 weeks, with no significant differences observed among treatment groups. Median GGT values decreased throughout the first 12 weeks, and greater reductions were observed in Croup 2 group vs. the placebo and Group 1. Median AST and ALT values were generally lower at day 1 of the second 12 weeks vs. baseline and AST reductions remained stable throughout the second 12 weeks. Levels of ALT decreased slightly. Median GGT values were lower at day 1 of the second 12 weeks vs. baseline and generally remained stable during the second 12 weeks.

At the first 12 weeks, a majority of patients in each treatment group (88% to 96%) reported at least 1 treatment-emergent adverse effect. Most treatment-emergent adverse effects were mild to moderate in severity but a higher proportion of patients in the treatment groups experienced severe treatment-emergent adverse effects than in the placebo group (Croup 1 28%; Croup 2 52%; placebo 17%).

In summary, trial demonstrated that treatment of PSC with multi-layered tablets comprising obeticholic acid (OCA) and ursodeoxycholic acid (UDCA) resulted in sustained reductions in serum ALP levels. The treatment regimen was generally safe and well tolerated with no evidence of worsening total bilirubin or other liver chemistries.

The technical contribution of the present disclosure resides in the following. Due to the selection and use of the new group of the additional markers, the authors were able to study the molecular processes in the liver and divide the patients by stages of PBC to apply the necessary treatment regimen and increase the effectiveness of the treatment up to 87% by using the highly compliant pharmaceutical composition that can apply to all stages of PBC and comprises UDCA and OCA together. The competition of ursodeoxycholic and obeticholic acids was also determined. In order to prevent the competition for the transporters, the new solid dosage form with a structural solution, such as the multilayer solid dosage form for the separate release of two acids, was developed.

The given embodiments of the present disclosure in no way limit it but are given only for a better understanding of its essence.

## Claims

1. A use of IL-6, Nf-kB, MCP-1/CCL2, and Bcl-2 markers for the diagnosis and adjustment of the treatment of primary biliary cholangitis.

2. The use according to claim 1, **characterised in that** TNF-α, NLRP3, and MDA markers are additionally used.

3. A pharmaceutical composition for the treatment of primary biliary cholangitis comprising ursodeoxycholic acid and at least one excipient, **characterised in that** it additionally comprises obeticholic acid.

4. The pharmaceutical composition according to claim 3, **characterised in that** it is made in the dosage form of an uncoated tablet, coated tablet, or capsule.

5. The pharmaceutical composition according to claim 3, **characterised in that** it has a modified release of obeticholic acid.

6. The pharmaceutical composition according to claim 3, **characterised in that** it has a modified release of ursodeoxycholic acid.

7. The pharmaceutical composition according to claim 3, **characterised in that** it is used for the treatment of primary biliary cholangitis with the values of the primary biliary cholangitis markers according to claim 1:
| | |
|---|---|
| IL-6 | - at least 3.0 pg/ml; |
| Nf-kB | - at least 14.0 pg/ml; |
| MCP-1/CCL2 | - at least 215 pg/ml; |
| Bcl-2 | - at least 0.24 U/I; |

8. The pharmaceutical composition according to claim 3, **characterised in that** it is used for the treatment of primary biliary cholangitis with the values of the primary biliary cholangitis markers according to claim 1:
| | |
|---|---|
| IL-6 | - at least 3.0 pg/ml; |
| Nf-kB | - at least 14.0 pg/ml; |
| MCP-1/CCL2 | - at least 215 pg/ml; |
| Bcl-2 | - at least 0.24 U/I; |
| TNF-α | - at least 0.14 hg/ml. |
| NLRP3 | - expression at least 1.2 times more than the control; |
| MDA | - at leas 4.3 nmol/ml. |

9. A method of determining the treatment regimen for primary biliary cholangitis, **characterised in that** it comprises the steps:
A) determining values of IL-6, Nf-kB, MCP-1/CCL2, and Bcl-2 markers in the patient sample;
B) comparing values of IL-6, Nf-kB, MCP-1/CCL2, and Bcl-2 markers with their control values;
C) determining treatment regimen for primary biliary cholangitis based on the values of IL-6, Nf-kB, MCP-1/CCL2, and Bcl-2 markers, wherein the said treatment regimen is a regimen based on stimulation of the FXR nuclear receptor, which leads to inhibition of the bile acid synthesis, as well as anti-inflammatory, anti-fibrotic and anti-apoptotic effects; and the usage of the pharmaceutical composition according to claim 3.

10. The method according to claim 9, **characterised in that** values of TNF-α, NLRP3, MDA markers are additionally determined in the patient sample.

11. A solid dosage form for the treatment of primary biliary cholangitis, **characterised in that** it comprises the pharmaceutical composition according to claim 3, wherein the mass ratio of ursodeoxycholic acid to obeticholic acid ranges from 2:1 to 1000: 1, and comprises the core comprising ursodeoxycholic acid and at least one layer comprising obeticholic acid, and is a dosage form with a modified release.

12. The solid dosage form according to claim 11, **characterised in that** the mass ratio of ursodeoxycholic acid to obeticholic acid ranges from 500: 1 to 1000: 1.

13. The solid dosage form according to claim 11, **characterised in that** the mass ratio of ursodeoxycholic acid to obeticholic acid ranges from 700: 1 to 1000: 1.

14. The solid dosage form according to claim 11, **characterised in that** it comprises three layers and the core.

15. The solid dosage form according to claim 11, **characterised in that** it comprises an outer layer, a second layer comprising obeticholic acid, a third layer, and a core comprising ursodeoxycholic acid.

16. The solid dosage form according to claim 11, **characterised in that** the outer layer is a coating or layer resistant to the action of gastric acid.

17. The solid dosage form according to claim 11, **characterised in that** the outer layer is nonresistant to the action of gastric acid.

18. The solid dosage form according to claim 11, **characterised in that** the second layer comprises obeticholic acid in an amount of 1-50 mg.

19. The solid dosage form according to claim 11, **characterised in that** the third layer is a STOP-layer with a delayed dissolution.

20. The solid dosage form according to claim 11, **characterised in that** the dissolution time of the STOP-layer is 1-2 hours.

21. The solid dosage form according to claim 11, **characterised in that** the core comprises ursodeoxycholic acid in an amount of 100-1000 mg.

22. The solid dosage form according to claim 11, **characterised in that** the core has a modified release of ursodeoxycholic acid with a duration within 1-6 hours.

23. The solid dosage form according to claim 11, **characterised in that** it comprises the outer layer being nonresistant to the action of gastric acid, the second layer comprising obeticholic acid, the third layer being enteric, and the core comprising ursodeoxycholic acid.

24. The solid dosage form according to claim 11, **characterised in that** it comprises the outer layer being resistant to the effect of gastric acid, the second layer comprising obeticholic acid, the STOP layer, and the core comprising ursodeoxycholic acid.

25. The solid dosage form according to claim 11, **characterised in that** it comprises the outer layer being resistant to the action of gastric acid, the second layer comprising obeticholic acid, the third layer with delayed dissolution, and the core comprising ursodeoxycholic acid, and it is used for the treatment of biliary cholangitis with the marker values:
IL-6 - at least 3.0 pg/ml;
Nf-kB - at least 14.0 pg/ml;
MCP-1/CCL2 - at least 215 pg/ml;
Bcl-2 - at least 0.24 U/I;

26. The solid dosage form according to claim 11, **characterised in that** it comprises the outer layer being resistant to the action of gastric acid, the second layer comprising obeticholic acid, the third layer with delayed dissolution, and the core comprising ursodeoxycholic acid, and it is used for the treatment of biliary cholangitis with the marker values:
IL-6 - at least 3.0 pg/ml;
Nf-kB - at least 14.0 pg/ml;
MCP-1/CCL2 - at least 215 pg/ml;
Bcl-2 - at least 0.24 U/I;
TNF-α - at least 0.14 hg/ml.
NLRP3 - expression is at least 1.2 times higher than control;
MDA - at least 4.3 nmol/ml.
